(19) Europäisches Patentamt
European Patent Office
Office européen des brevets

(11) Publication number: **0 403 398 B1**

(12) # EUROPEAN PATENT SPECIFICATION

(45) Date of publication of patent specification: **30.11.94**

(21) Application number: **90401735.7**

(22) Date of filing: **15.06.90**

(51) Int. Cl.[5]: **C07D 213/59**, C07D 215/14, C07D 217/14, C07D 237/08, C07D 239/26, C07D 241/12, C07D 209/20, C07D 277/30, C07C 327/46, C07D 495/04, A61K 31/44, //A61K31/47, A61K31/495,A61K31/425, A61K31/40,A61K31/16, A61K7/06,(C07D495/04,333:00, 221:00)

(54) **New thioformamide derivatives.**

(30) Priority: **16.06.89 GB 8913864**
**16.06.89 GB 8913863**

(43) Date of publication of application:
**19.12.90 Bulletin 90/51**

(45) Publication of the grant of the patent:
**30.11.94 Bulletin 94/48**

(84) Designated Contracting States:
**AT BE CH DE DK ES FR GB GR IT LI LU NL SE**

(56) References cited:
**EP-A- 0 321 273**
**EP-A- 0 321 274**

(73) Proprietor: **RHONE-POULENC SANTE**
**20, avenue Raymond Aron**
**F-92160 Antony (FR)**

(72) Inventor: **Hart, Terance William**
**17 Rowhedge,**
**Hutton**
**Brentwood, Essex (GB)**
Inventor: **Vacher, Bernard Yvon Jack**
**139 Amesbury Road**
**Dagenham, Essex (GB)**
Inventor: **Walsh, Roger John Aitchison**
**38 The Paddocks**
**Rayleigh, Essex (GB)**

**EP 0 403 398 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS vol. 82, no. 19, 12 May 1975, page 561, abstract no. 225377c, Columbus, Ohio, US; H. SINGH et al.: "Synthesis of heterocyclics via enamines. II. Reactions of cyclohex-1-enylaniline-morpholine, and -piperidine with 1,1 -dimethyl-3-oxobutyl isothiocyanate" & Aust. J. Chem. 1975, vol. 28, no. 1, pages 143-149**

(74) Representative: **Pilard, Jacques et al**
**RHONE-POULENC SANTE,**
**Service Brevets,**
**20 Avenue Raymond Aron**
**F-92165 Antony Cédex (FR)**

## Description

This invention relates to new therapeutically useful thioformamide derivatives, to processes for their preparation and to pharmaceutical compositions containing them.

The new thioformamide derivatives of the present invention are those compounds of the general formula (I) hereinafter depicted, wherein R represents a straight- or branched-chain alkyl radical containing from 1 to 4 carbon atoms, A represents an aromatic heterocyclic radical containing one or two nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl and thieno[3,2-b]pyridin-6-yl, or represents a phenyl group substituted in the 3 and/or 5 position with a cyano, nitro, trifluoromethyl, carbamoyl, carboxy, $C_{2-5}$-alkanoyl, $C_{2-5}$-alkoxycarbonyl or $C_{1-4}$-alkylsulphonyl group or a fluorine, chlorine or bromine atom, and optionally further substituted with halogen atom(s), $C_{1-4}$-alkyl or $C_{6-12}$-aryl (e.g. phenyl) group(s), or A may be a phenyl group unsubstututed or substituted with halogen atom(s), $C_{1-4}$-alkyl or $C_{6-12}$-aryl group(s) or with substituents which together form a fused ring and Y represents a valency bond or a methylene or ethylene radical, $R^2$ represents a hydrogen atom, an alkyl, cycloalkyl, cycloalkylalkyl, aryl, aralkyl, aryloxyalkyl, aromatic heterocyclylalkyl or aromatic heterocyclyloxyalkyl group, optionally substituted by one or more halogen atoms or a group BC(=O)- in which B represents an alkyl, aryl, or aromatic heterocyclic group, optionally substituted by one or more halogen atoms, or B represents a benzyl, phenethyl, 1-naphthylmethyl, 2-naphthylmethyl or pyrid-3-ylmethyl radical, n represents 0 or 1, and when n represents 0 $R^1$ may represent a hydrogen atom, an alkyl, cycloalkyl, cycloalkylalkyl, aryl, aralkyl, aryloxyakyl, aromatic heterocyclylalkyl or aromatic heterocyclyloxyalkyl group, optionally substituted by one or more halogen atoms or a group BC(=O)- or $BSO_2$-, and when n represents 1 $R^1$ represents a straight- or branched-chain alkyl radical containing from 1 to 4 carbon atoms which is unsubstituted or substituted by one or more substituents selected from $C_{2-4}$-alkenyl, carboxy, $C_{2-5}$-alkoxycarbonyl, hydroxy, $C_{1-4}$-alkoxy, carbamoyl (unsubstituted or substituted by one or two $C_{1-4}$-alkyl groups), amino, $C_{1-4}$-alkylamino and di-$C_{1-4}$-alkylamino groups or represents a benzyl, phenethyl, 1-naphthylmethyl, 2-naphthylmethyl or pyrid-3-ylmethyl radical each of which may be substituted on the ring by one or more halogen atoms or hydroxy, $C_{1-4}$-alkyl, $C_{1-4}$-alkoxy (alkoxy being unsubstituted or substituted as defined for alkyl groups represented by $R^1$), cyano, nitro, trifluoromethyl, carboxy, $C_{1-4}$-alkylamino, $C_{2-5}$-alkanoylamino or $C_{2-5}$-alkoxycarbonyl groups, the aryl groups and moities in the definition of $R^1$, $R^2$ and B being selected from phenyl and naphthyl optionally substituted by one or more halogen atoms and the aromatic heterocyclic groups and moities in the definition of $R^1$, $R^2$ and B being selected from pyrid-3-yl and pharmaceutically acceptable salts thereof.

Preferably A represents 3-pyridyl, 6-chloropyrid-3-yl, 5-bromopyrid-3-yl, 3-quinolyl, 4-isoquinolyl or 5-pyrimidyl, or phenyl substituted in the 3 and/or 5 position with an electron-withdrawing group for example a cyano, nitro, trifluoromethyl, carbamoyl, carboxy, $C_{2-5}$-alkanoyl, $C_{2-5}$-alkoxycarbonyl or $C_{1-4}$-alkylsulphonyl or a fluorine, chlorine or bromine atom, and optionally further substituted with halogen atom(s), $C_{1-4}$-alkyl or $C_{6-12}$-aryl (e.g. phenyl) group(s), or the phenyl group A may be substituted with halogen atom(s), $C_{1-4}$-alkyl or $C_{6-12}$-aryl (e.g. phenyl) group(s) or with substituents which together form a fused ring, for example a 2-naphthyl group.

The group A represents, for example, the phenyl, 4-chlorophenyl, 3-trifluoromethylphenyl, 3-nitrophenyl, 3,4-dichlorophenyl or 2-naphthyl group.

Preferably B represents a phenyl or pyrid-3-yl group or a straight- or branched-chain alkyl group (containing from 1 to 6 carbon atoms), each of which being optionally substituted by one or more substituents selected from halogen atoms, or B represents a benzyl, phenethyl, 1-naphthylmethyl, 2-naphthylmethyl or pyrid-3-ylmethyl radical.

The group B represents, for example, the phenyl, 4-fluorophenyl, 3,4-difluorophenyl, pyrid-3-yl, methyl, butyl, benzyl or phenethyl.

Preferably $R^2$ represents the hydrogen atom. Amino groups $R^1R^2N$- that may be mentioned are 1-phenylthylamino, 1-(1-naphthyl)ethylamino, 1-cyclohexylethylamino or 1-(pyrid-3-yl)ethylamino.

The presence of an amino group on the ring creates an asymmetry in the molecule which, in association with the adjacent asymmetric carbon atom, leads to 4 stereoisomers which, optionally, can be separated into 2 racemic pairs. The racemic pair and its enantiomers of the general formula (II) in which R, A and Y are as hereinbefore defined, i.e. the compounds in which the amino group is in the trans position relative to the group -CSNHR, are preferred.

Furthermore, in certain cases the substituents R, $R^1$ and $R^2$ contribute to stereoisomerism. All such forms are embraced by the present invention.

Especially important compounds of the present invention include those wherein at least one of the symbols has a value selected from the following:-

(i) R represents methyl;

(ii) A represents3-pyridyl;

(iii) Y represents methylene;

(iv) B represents lower alkyl, e.g. methyl, or optionally substituted phenyl;

(v) $R^2$ represents hydrogen; and

(vi) $R^1$ represents hydrogen, lower alkyl, e.g. methyl or ethyl, optionally carrying a substituent selected from aryl, preferably naphthyl or optionally substituted phenyl (the said optional substituents on phenyl being preferably one or more halogen, preferably fluorine, atoms), or 3-pyridyl, cycloalkyl, e.g. cyclohexyl, and hydroxy groups, or represents a lower alkanoyl, preferably acetyl, or aroyl, preferably benzoyl, or arylsulphonyl, preferably phenylsulphonyl, group;

the other symbols being as hereinbefore defined, and their pharmaceutically acceptable salts.

Particularly important compounds of the present invention include the following:-

AA (±)-trans-2-benzyloxyamino-N-methyl-1-(3-pyridyl)cyclohexanecarbothioamide
AB (±)-trans-2-methoxyamino-N-methyl-1-(3-pyridyl)cyclohexanecarbothioamide
AC (±)-trans-2-(4-fluorobenzyloxyamino)-N-methyl-1-(3-pyridyl)cyclohexanecarbothioamide
AD (1S,2R)-2-(2,3,4,5,6-pentafluorobenzyloxyamino)-N-methyl-1-(3-pyridyl)cyclohexanecarbothioamide
AE (±)-trans-2-(2-hydroxyethoxyamino-N-methyl-1-(3-pyridyl)cyclohexanecarbothioamide
BA (±)-trans-2-amino-N-methyl-1-(3-pyridyl)cyclohexanecarbothioamide
BB (±)-trans-2-benzamido-N-methyl-1-(3-pyridyl)cyclohexanecarbothioamide
BC (±)-trans-2-acetamido-N-methyl-1-(3-pyridyl)cyclohexanecarbothioamide
BD (2R,1S)-2-[(R)-1-phenylethylamino]-1-(3-pyridyl)cyclohexane-N-methylcarbothioamide
BE (2R,1S)-2-[(R)-1-(1-naphthyl)ethylamino]-1-(3-pyridyl)cyclohexane-N-methylcarbothioamide
BF (2R,1S)-2-[(R)-1-cyclohexylethylamino]-1-(3-pyridyl)cyclohexane-N-methylcarbothioamide
BG (±)-(2R,1S)-2-[(R)-1-(4-fluorophenyl)ethylamino]-1-(3-pyridyl)cyclohexane-N-methylcarbothioamide
CA (±)-trans-N-methyl-2-phenylsulphonamido-1-(3-pyridyl)cyclohexanecarbothioamide
CB (±)-trans-N-methyl-2-phenylamino-1-(3-pyridyl)cyclohexanecarbothioamide
CC (±)-trans-N-methyl-1-benzylamino-2-(3-pyridyl)cyclohexanecarbothioamide
CD (-)-(1R,2S)-N-methyl-2-(3-pyridyl)methylamino-1-(3-pyridyl)cyclohexanecarbothioamide

The letters AA to CD are allocated to the compounds for easy reference later in the specification, e.g. in the Table and in the Examples.

The compounds have valuable pharmacological properties, in particular properties which are indicative of utility in the treatment and/or propylaxis of disorders associated with:-

(1) vascular smooth muscle contraction including hypertension and other cardiovascular disorders such as congestive heart failure, and conditions associated with tissue ischaemia such as angina, peripheral vascular disease and cerebrovascular disease;

(2) respiratory smooth muscle contraction including reversible airways obstruction and asthma;

(3) contraction of smooth muscle of gastro-intestinal tract, urinary bladder and uterus, including peptic ulcers, irritable bowel syndrome and diverticular disease; irritable bladder syndrome; and premature labour.

The compounds also have utility in the inhibition of head hair loss associated with male pattern baldness, by topical application.

For example, compounds of general formula (I) were submitted to:-

Vaso-relaxant Activity Tests.

The test methods used were adapted from those described by Winslow et al [Eur.J.Pharmacol., 131, 219-228 (1986)] and Karaki [J.Pharmacol. Methods, 18, 1-21 (1987)] for differentiating vaso-relaxant activity.

Test A : Activity against contractions induced by low $K^+$ concentrations in the isolated rat aorta

Thoracic aorta was removed from rats and transverse strips, denuded of endothelium, were suspended in a bath containing Krebs solution. The tension was recorded and a contraction induced by addition of 20mM $K^+$ (potassium ion) to the bathing solution. The test compound was added to the bath as a solution in increasing cumulative concentration. The concentration in the bathing solution of the test compound which reduced the $K^+$-induced contraction by 90% was determined and expressed in $\mu$M as the effective concentration ($EC_{90}$), given in Table I.

Table I

| Compound | Activity Test A $EC_{90}$ μM |
|---|---|
| AA | 0.003 |
| AB | 0.3 |
| BD | 0.00003 |
| BE | 0.003 |

The compounds of general formula (I) and their intermediates can be prepared by the application or adaptation of known methods, for example as hereinafter identified.

By the term "known methods" as used in this specification is meant methods heretofore used or described in the literature, for example in the specifications of European Patent Applications Nos. 321274A and 321273A and their United States equivalents USSN 07/285219 and 07/285114.

According to a feature of the present invention, the compounds of general formula (I) wherein $R^2$ represents the hydrogen atom may be prepared by the reduction of a compound of general formula (III) wherein $R^{1'}$ is as hereinbefore defined for $R^1$ or, when n is 1, $R^{1'}$ may represent hydrogen.

The reduction is effected using a reducing agent, e.g lithium aluminium hydride, sodium borohydride, sodium cyanoborohydride or borane, in an inert organic solvent optionally under inert atmosphere, at 0-50 °C.

According to a feature of the present invention, the compounds of general formula (I) wherein one or both of $R^1$ and $R^2$ represents an optionally substituted alkyl, cycloalkyl, cycloalkylalkyl, aryl, aralkyl, aryloxyalkyl, aromatic heterocyclylalkyl or aromatic heterocyclyloxyalkyl group may be prepared by the reaction of a compound of general formula (I) wherein at least one of $R^1$ and $R^2$ represents the hydrogen atom with the corresponding alkyl, cycloalkyl, cycloalkylalkyl, aryl, aralkyl, aryloxyalkyl, aromatic heterocyclylnalkyl or aromatic heterocyclyloxyalkyl halide in the absence or presence of an inert organic solvent, for example an aromatic hydrocarbon, e.g. benzene or toluene, chloroform, dichloromethane, tetrahydrofuran or dimethylformamide, at a temperature from 0 °C up to the reflux temperature of the reaction mixture.

According to a feature of the present invention, the compounds of general formula (I) wherein one or both of $R^1$ and $R^2$ represents a group BC(=O)- as hereinbefore defined may be prepared by the reaction of a compound of general formula (I) wherein at least one of $R^1$ and $R^2$ represents a hydrogen atom with an acid halide of a compound of general formula

BCOOH (IV)

or an reactive acid anhydride thereof.

The reaction is effected, optionally in the presence of an acid acceptor, for example a tertiary amine, e.g. triethylamine, or an inorganic base, e.g. sodium bicarbonate, preferably in the presence of an anhydrous inert organic solvent, e.g. chloroform or acetone, and preferably at a temperature of from -30 °C to +30 °C.

According to a feature of the present invention, the compounds of general formula (I) wherein $R^1$ represents a group $BSO_2$- as hereinbefore defined may be prepared by the reaction of a compound of general formula (I) wherein n is 0 and $R^1$ represents hydrogen atom with a sulphonyl halide of the general formula:-

$BSO_2X^1$ (V)

wherein B is as hereinbefore defined and $X^1$ represents a halogen atom, preferably chlorine.

The reaction is optionally effected in the presence of an acid acceptor, for example a tertiary amine, e.g. pyridine, and preferably at a temperature of from -30 °C to +30 °C, preferably at or near 0 °C.

The thioformamide derivatives of general formula (I) obtained by the aforedescribed processes can be purified by the usual physical methods, in particular crystallisation and chromatography, especially to resolve mixtures of enantiomers using a chiral column.

By the term "pharmaceutically acceptable salts" as used in this specification is meant salts the anions or cations of which are relatively innocuous to the animal organism when used in therapeutic doses so that the beneficial pharmaceutical properties of the parent compounds of general formula (I) capable of forming salts are not vitiated by side-effects ascribable to those anions or cations.

As well as being useful in themselves as active compounds, acid addition salts of the compounds of general formula (I) capable of forming such salts are useful for the purposes of purification of the parent compounds of general formula (I), for example by exploitation of the solubility differences between the salts and the parent compounds, by techniques well known to those skilled in art. The parent compounds of general formula (I) can be regenerated from their acid addition salts by known methods, for example by treatment with an alkali, e.g. aqueous sodium bicarbonate solution or aqueous ammonia solution.

Suitable acid addition salts for use in pharmaceuticals may be selected from salts derived from inorganic acids, for example hydrochlorides, hydrobromides, phosphates, sulphates and nitrates, and organic acids, for example oxalates, lactates, tartrates, acetates, salicylates, citrates, propionates, succinates, fumarates, maleates, methylene-bis-$\beta$-hydroxynaphthoates, gentisates and di-p-toluoyltartrates.

As well as being useful in themselves as active compounds, salts of the compounds of general formula (I) capable of forming salts with bases are useful for the purposes of purification of the parent compounds of general formula (I), for example by exploitation of the solubility differences between the salts and the parent compounds, by techniques well known to those skilled in the art.

Suitable salts with bases include alkali metal (e.g. sodium and potassium), alkaline earth metal (e.g. calcium and magnesium), ammonium and amine (e.g. diethanolamine, triethanolamine, octylamine, morpholine and dioctylmethylamine) salts.

In this specification reference to compounds of formula (I) is intended to include reference to their pharmaceutically acceptable salts, where the context so permits.

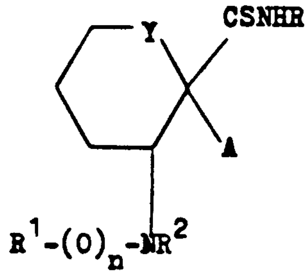

I

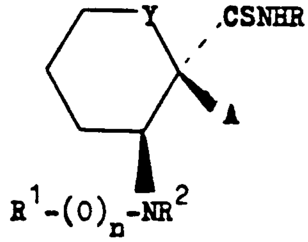

II

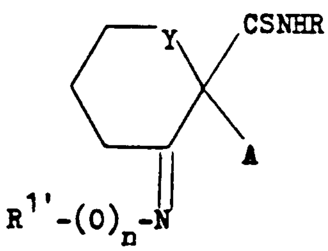

III

The following Examples illustrate the preparation of compounds according to the present invention.

Unless stated otherwise, all the nuclear magnetic resonance (NMR) spectra were recorded at 200 MHz in deuterochloroform; the chemical shifts are expressed in ppm relative to the tetramethylsilane signal. The abbreviations used in the following text are as follows:

s = singlet, d = doublet, t = triplet, q = quartet, m = multiplet, c = unresolved bands and b = broad.

EXAMPLE 1

Compond AA

A stirred solution of (±)-2-benzyloxyimino-N-methyl-1-(3-pyridyl)cyclohexanecarbothioamide (2.5g) in dry tetrahydrofuran (50ml) under argon was treated portionwise with lithium aluminium hydride (0.305g) at 25° and stirred at that temperature for 3.5 hours. The green suspension was cooled in ice and treated dropwise with water (75ml) at 5°. The mixture was extracted with chloroform (3x75ml), the combined extracts dried (magnesium sulphate) and evaporated. The residue was subjected to flash chromatography on silica gel eluting with chloroform/methanol : 99/1 followed by washing with hexane to give (±)-trans-2-benzyloxyamino-N-methyl-1-(3-pyridyl)cyclohexanecarbothioamide, a colourless solid (1.1g, 43%), m.p. 136-137°. Found: C, 67.6; H, 7.02; N, 11.7%; $C_{20}H_{25}N_3OS$ requires C, 67.6; H, 7.09; N, 11.8%.

EXAMPLE 2

Compound AB

Prepared from (±)-2-methoxyimino-N-methyl-1-(3-pyridyl)cyclohexanecarbothioamide (2.5g) as described in Example 1. The crude product was subjected to flash chromatography on silica gel eluting with chloroform/methanol : 98.5/1.5, washed with hexane to give (±)-trans-2-methoxyamino-N-methyl-1-(3-pyridyl)cyclohexanecarbothioamide, a colourless solid (1.0g, 40%), m.p. 165-170°.

EXAMPLE 3

Compound AC

Prepared from (±)-2-(4-fluorobenzyloxyimino)-N-methyl-1-(3-pyridyl)cyclohexanecarbothioamide (4.6g) as described in Example 1. The crude product was subjected to flash chromatography on silica gel eluting with chloroform/methanol : 98/2, washed with pentane to give (±)-trans-2-(4-fluorobenzyloxyamino)-N-methyl-1-(3-pyridyl)cyclohexanecarbothioamide, a colourless solid (0.9g, 19%), m.p. 128.130°. Found: C, 64.6; H, 6.6; N, 11.2%; $C_{20}H_{24}FN_3OS$ requires C, 64.3; H, 6.48; N, 11.0%.

EXAMPLE 4

Compound AD

Prepared from (-)-(S)-2-(2,3,4,5,6-pentafluorobenzyloxyimino)-N-methyl-1-(3-pyridyl)-cyclohexanecarbothioamide (0.49g) as described in Example 1. The crude product was subjected to flash chromatography on silica gel eluting with chloroform/methanol : 98.5/1.5 to give (1S,2R)-2-(2,3,4,5,6-pentafluorobenzyloxyamino)-N-methyl-1-(3-pyridyl)cyclohexanecarbothioamide, a yellow solid (0.34g, 69%, m.p. 57-60°. Found: C. 53.7; H, 4.37; N, 9.5%; $C_{20}H_{20}F_5N_3OS$ requires C, 53.9; H, 4.53; N, 9.43%.

EXAMPLE 5

Compound AE

A stirred solution of (±)-2-(2-hydroxyethoxyimino)-N-methyl-1-(3-pyridyl)cyclohexanecarbothioamide (2.5g) in dry tetrahydrofuran (45ml) under argon was treated portionwise with lithium aluminium hydride (0.35g), the temperature rising to 43°. The mixture was then stirred at 25° for 3 hours. It was then treated with a further quantity oflithium aluminium hydride (0.34g) and stirred at 25° for 3 hours.

The mixture was cooled in an ice bath and treated dropwise with water (80ml), extracted with ethyl acetate (3x80ml), and the combined extracts were dried and evaporated. The residue was subjected to flash chromatography on silica gel eluting with chloroform/methanol: 95/5, washed with diethyl ether, to give (±)-trans-2-(2-hydroxyethoxyamino-N-methyl-1-(3-pyridyl)cyclohexanecarbothioamide, a colourless solid (0.43g, 17%), m.p. 142-145°. Found: C, 58.0; H, 7.3; N, 13.5. $C_{15}H_{23}N_3O_2S$ requires C, 58.2; H, 7.49; N. 13.6.

EXAMPLE 6

Compound BA

A solution of (±)-2-hydroxyimino-N-methyl-1-(3-pyridyl)cyclohexanecarbothioamide (13.2g) in dry tetrahydrofuran (500ml) was added dropwise to a stirred suspension of lithium aluminium hydride (7.66g) in tetrahydrofuran (250ml) under argon during 30 minutes. The mixture was heated at reflux for 1.5 hours, and then it was cooled to 5° and treated dropwise with water (750ml). The mixture was extracted with chloroform (7 x 350ml). The combined extracts were dried (magnesium sulphate) and evaporated. The residue was subjected to flash chromatography on silica gel, eluting with ethyl acetate/methanol/triethylamine: 90/18/5, washed with diethyl ether, to give (±)-trans-2-amino-N-methyl-1-(3-pyridyl)cyclohexanecarbothioamide,a colourless solid (2.3g, 19%), m.p. 184-188°.

EXAMPLE 7

Compound BB

A solution of (±)-trans-2-amino-N-methyl-1-(3-pyridyl)cyclohexanecarbothioamide (0.51g) in dry pyridine (5.3ml) was cooled to 0° and treated dropwise with benzoyl chloride (0.29g) during 16 minutes. Stirring was continued at 0° for 2.5 hours.

The solution was concentrated in vacuo (40°/0.3 mm Hg) and the residue partitioned between chloroform (10ml) and 2M aqueous sodium carbonate solution. The chloroform layer was washed with water (10ml), dried (magnesium sulphate) and evaporated. The residue was subjected to flash chromatography on silica gel, eluting with chloroform/methanol : 95/5, washed with diethyl ether to give (±)-trans-2-benzamido-N-methyl-1-(3-pyridyl)cyclohexanecarbothioamide, a yellow solid (0.15g, 29%), m.p. 125-130°.

EXAMPLE 8

Compound BC

Prepared from (±)-trans-2-amino-N-methyl-1-(3-pyridyl)cyclohexanecarbothioamide (1.0g) and acetyl chloride (0.32g) as described in Example 2. The crude product was subjected to flash chromatography on silica gel eluting with methylene chloride/methanol: 92/8, washed with diethyl ether to give (±)-trans-2-acetamido-N-methyl-1-(3-pyridyl)cyclohexanecarbothioamide, a colourless solid (0.28g, 24%), m.p. 226-229°.

EXAMPLE 9

Compounds BD, BE, BF and BG

A mixture of 2-(3-pyridyl)cyclohexanone (10g, 57 mmol), (R)-1-phenylethylamine (8.1ml, 68 mmol) and p-toluenesulphonic acid hydrate (0.11g, 0.57 mmol) in anhydrous toluene (85ml) was heated at reflux in a Dean-Stark apparatus for 3 hours under an argon atmosphere.

The cooled solution was concentrated in vacuo at 14mm and 50°C and then at 0.1mm and 40°C to furnish 2-(3-pyridyl)-1-[(R)-1-phenylethylimino]cyclohexane as a crude red oil (18g) which was used in the next stage without further purification.

A stirred solution of this crude 2-(3-pyridyl)-1-[(R)-1-phenylethylimino]cyclohexane (18g) in anhydrous tetrahydrofuran (80ml) at -78°C was treated dropwise with n-butyllithium (1.6M in hexane) (39.5ml, 62.8 mmol) over 1 hour.

The resulting deep red solution was stirred at -60°C for a further 1 hour then cooled to -78°C. A solution of methyl isothiocyanate (5g, 68.6 mmol) in tetrahydrofuran (8ml) was added dropwise over a period of 10 minutes to the reaction mixture, which was then allowed to warm to 0°C over a period of 1 hour. After an extra period of 30 minutes at 0°C the resulting mixture was poured into a cold solution of ammonium chloride (20% w/w/) (100ml). The organic layer was removed and the aqueous layer extracted with diethyl ether (2 x 100ml). The combined organic fractions were washed successively with water (100ml) and brine (100ml) then dried over magnesium sulphate. Concentration in vacuo gave crude 2-[(R)-1-phenylethylimino]-(S)-1-(3-pyridyl)cyclohexane-N-methylcarbothioamide as an orange oil (23g), which was used in the next stage without further purification.

A solution of crude 2-[(R)-1-phenylethylimino]-(S)-1-(3-pyridyl)cyclohexane-N-methylcarbothioamide (23g) in anhydrous methanol (70ml) at 0°C under an argon atmosphere was treated with sodium cyanoborohydride (9g, 143 mmol). Glacial acetic acid (9.8ml, 170 mmol) was then added dropwise to the mixture over a period of 15 minutes at 0°C. The reaction mixture was then allowed to warm to 20°C over 1 hour and stirred for a further 30 minutes at 20°C. Aqueous sodium carbonate (1.5M) (115ml, 173 mmol) was then slowly added to the reaction mixture which was then extracted with chloroform (2x200ml). The combined organic extracts were washed successively with water (200ml) and brine (50ml) then dried over sodium sulphate. Concentration under reduced pressure at 30°C and 14mmHg gave a crude red oil which was recrystallised from ethyl acetate to give (2R,1S)-2-[(R)-1-phenylethylamino]-1-(3-pyridyl)cyclohexane-N-methylcarbothioamide (6.4g, 18.1 mmol, 32% overall yield) m.p. 165-166°C found; C, 71.1%; H, 7.7%; N, 11.9%, S, 9.1%. $C_{21}H_{27}N_3S$ requires C, 71.3%, H, 7.7%, N, 11.9%, S, 9.1%. $[\alpha]_D^{25}$ = + 61° (C = 0.47, $CHCl_3$) 'H. N.M.R. 1.05-1.805 (m,7H), 1.35 (d,3H), 2.35 (ddd,1H); 2.7 (dd,1H), 3.2 (d,3H), 3.2 (dd,1H), 3.7 (q,1H), 7.2-7.4 (m,6H), 8.0 (ddd,1H), 8.5 (dd,1H), 8.9 (d,1H), 9.8 (broad s,1H).

In a similar manner the following were prepared:-

* (2R,1S)-2-[(R)-1-(1-naphthyl)ethylamino]-1-(3-pyridyl)cyclohexane-N-methylcarbothioamide (recrystallised from ethyl acetate) (47% overall yield) m.p. 204-205 °C. Found: C, 74.4%; H, 7.4%; N, 10.3%; S, 7.8%. $C_{25}H_{29}N_3S$ requires C, 74.4%; H, 7.3%; N, 10.4%; S, 7.9%. $[\alpha]_D^{25}$ = -70 ° (C = 0.5, $CHCl_3$) 'H N.M.R. 1.1 - 1.65 (m,6H), 1.4 (d,3H), 1.7 (bs,1H); 1.9 (ddd,1H), 2.4 (ddd,1H), 2.6 (dd,1H), 3.2 (d,3H), 3.5 (dd,1H), 4.6 (q,1H), 7.3 (dd,1H), 7.4 - 7.6 (m,4H), 7.8 (d,1H), 7.9 (dd,1H), 8.1 (m,2H), 8.5 (dd,1H), 9.0 (d,1H), 9.5 (broad s,1H),

* using (R)-1-(1-naphthyl)ethylamine as initial starting material;

** (2R,1S)-2-[(R)-1-cyclohexylethylamino)-1-(3-pyridyl)cyclohexane-N-methylcarbothioamide (subjected to flash chromatography over silica gel, eluting with a 55:40.5 mixture of chloroform, diethyl ether and ethanol, and then recrystallised from ethyl acetate) (17% overall yield) m.p. 138-139 °C. Found: C, 70.1%; H, 9.5%; N, 11.6%; S, 8.9%. $C_{21}H_{33}N_3S$requires C, 70.1, H, 9.3; N, 11.7; S, 8.9. $[\alpha]_D^{25}$ = +30 ° (C = 0.43, $CHCl_3$); NMR :- 0.98 (d,3H), 0.98 - 1.6 (m,9H), 1.6 - 1.9 (m,8H), 2.0-2.2 (m,2H), 2.6 (c,1H), 3.0 (dd,1H), 3.06 (dd, 1H), 3.1 (d,3H), 7.3 (dd,1H), 7.9 (ddd,1H); 8.3 (dd,1H), 8.9 (d,1H), 10.0 (broad s,1H).

** using (R)-1-cyclohexylethylamine as initial starting material; and

*** (±)-(2R,1S)-1-[(R)-1-(4-fluorophenyl)ethylamino]-2-(3-pyridyl)cyclohexane-N-methylcarbothioamide (subjected to flash column chromatography over silica gel, eluting with a 55:40.5 mixture of chloroform, diethyl ether and ethanol, and then recrystallised from ethyl acetate) (10% overall yield) m.p. 130-135 °C. Found: C, 67.8; H, 7.2; N, 11.3; S, 8.6%. $C_{21}H_{26}FN_3S$ requires C, 67.9; H, 7.1; N, 11.3; S, 8.6%;; NMR :- 1.0-1.2 (m,1H), 1.2-1.8 (c,9H); 2.35 (m,H), 2.5-2.6 (m,1H), 3.1 (d,3H), 3.2-3.3 (dd,1H), 3.6 (q,1H), 8.5 (m,1H), 8.9 (m,1H), 9.2-9.3 (broad singlet, 1H).

*** using (±)-1-(4-fluorophenyl)ethylamine as initial starting material.

EXAMPLE 10

Compound CA

A stirred solution of (±)-trans-2-amino-N-methyl-1-(3-pyridyl)cyclohexanecarbothioamide (0.5g) in dry pyridine (5ml) was treated with benzenesulphonyl chloride (35g) dropwise at 0 °C. Stirring was continued at 0 °C for 1.5 hours. The solution was evaporated in vacuo and the residue was partitioned between water (10ml) and chloroform (10ml). The organic phase was washed with water (10ml) dried, and evaporated. The resulting red oil was subjected to flash chromatography on silica gel, eluting with a mixture of methylene chloride and methanol (96:4v/v) to give, after trituration with diethyl ether, (±)-trans-N-methyl-2-phenylsulphonamido-1-(3-pyridyl)cyclohexanecarbothioamide (0.16g), in the form of a pale orange solid, m.p. 254-255 °C. [NMR (400MHz):- 1.3-2.4 (8H,m), 3.0 (3H,d), 4.7(1H,m), 5.2 (1H,d), 7.1 (1H,q), 7.5 (2H,t), 7.57 (1H,m), 7.62 (1H,br), 7.7 (2H,m), 7.9 (1H,m), 8.4 (1H,m), 8.5 (1H,d)].

EXAMPLE 11

Compounds CB, CC and CD

A stirred suspension of (±)-N-methyl-2-oxo-1-(3-pyridyl)cyclohexanecarbothioamide (2.5g) and aniline (3.72g) in anhydrous dichloroethane (30ml) under an argon atmosphere was treated dropwise at 25 °C with titanium tetrachloride (0.8ml). The reaction mixture was stirred at 25 °C until completion of the reaction (as judged by TLC analysis), and then it was poured into a solution of sodium bicarbonate (16.8g) in water (100ml) and stirred for 15 minutes. The mixture was filtered through a pad of celite and the aqueous layer was extracted with dichloromethane (3x75ml). The combined organic solution was washed with aqueous sodium bicarbonate solution (100ml; 10%w/w), with water (50ml) and with saturated brine (100ml), and then it was dried over sodium sulphate and concentrated in vacuo, to give N-methyl-2-phenylimino-1-(3-pyridyl)-cyclohexanecarbothioamide in the form of a crude oil.

A stirred solution of this crude N-methyl-2-phenylimino-1-(3-pyridyl)cyclohexanecarbothioamide in dichloromethane (15ml), cooled at 0 °C under an argon atmosphere, was treated with sodium cyanoborohydride (1.6g), in one portion. Dry methanol (30ml) was then added, followed by a dropwise addition of glacial acetic acid (3ml) The temperature was allowed to rise to 25 °C and then the reaction mixture was poured into saturated aqueous sodium bicarbonate solution (150ml). The aqueous phase was extracted with dichloromethane (3 x 100ml) and the combined organic extracts were washed with saturated aqueous sodium bicarbonate solution (100ml), with water (50ml) and then with saturated brine (100ml), dried over

sodium sulphate and concentrated in vacuo, to give a crude oil which was crystallised form a mixture of diethyl ether and ethyl acetate, to give (±)-trans-N-methyl-2-phenylamino-1-(3-pyridyl)-cyclohexanecarbothioamide (1.9g, 5.83), m.p. 195-196 °C . [NMR :- 1.42-1.67 (m,4H); 1.80-1.90 (m,1H); 2.11-2.18 (m,1H); 2.31-2.37 (m, 1H); 2.62-2.70 (m,1H); 3.00 (d,3H); 3.61 (bd,1H); 4.49 (bs,1H); 6.62 (dd,2H); 6.74 (ddt,1H); 7.13 (dt,2H); 7.23 (dd,1H); 7.90 (dt,1H); 8.42 (bs,1H); 8.46 (dd,1H); 8.75 (d,1H). Elemental analysis:- C,70.40;H,7.20; N,12.90;S,9.70%; calculated:- C,70.10;H,7.10;N,12.90; S,9.80%].

By proceeding in a similar manner, but replacing the aniline by the appropriate quantities of benzylamine and 3-pyridylmethylamine respectively, there were prepared:-

(±)-trans-N-methyl-1-benzylamino-2-(3-pyridyl)cyclohexanecarbothioamide, m.p. 125-126 °C (from a mixture of diisopropyl ether and diethyl ether) [NMR :-1.25-1.66 (m, 5H); 1.73-1.79 (m, 1H); 2.11-2.18 (m, 1H); 2.25 (dt, 1H); 2.73-2.80 (m, 1H); 3.04 (d, 3H); 3.33 (dd, 1H), 3.75 (dd, 2H); 7.24-7.37 (m, 6H); 8.05 (dt, 1H); 8.48 (dd, 1H); 8.91 (d, 1H) 9.00 (bs, 1H);

Calculated:- C, 70.70; H, 7.40; N, 12.40; S, 9.50%;

Found:- C, 70.50; H, 7.50; N, 12.30; S, 9.50%], and (-)-(1R,2S)-N-methyl-2-(3-pyridyl)methylamino-1-(3-pyridyl)cyclohexanecarbothioamide, m.p. 141-142 °C (from ethyl acetate) [NMR :- 1.29-1.63 (m,6H), 1.68-1.75 (m,1H); 2.08-2.14 (m, 1H); 2.42 (dt, 1H); 2.53-2.59 (m, 1H) 3.06 (d, 3H); 3.59 (dd, 1H); 3.75 (dd, 2H); 7.24-7.29 (m, 2H); 7.55 (dt, 1H); 8.10 (dd, 1H); 8.48-8.52 (m, 2H); 8.60 (bs, 1H); 8.93 (d, 1H);

Calculated:- C, 67.00; H, 7.10; N, 16.45; S, 9.40%;

Found:- C, 66.80; H, 7.20; N, 16.30; S, 9.60%;

$[\alpha]_D^{25}$ = - 66.9 ° (c = 0.538, $CHCl_3$)].

REFERENCE EXAMPLE 1

A solution of (±)-N-methyl-2-oxo-(3-pyridyl)cyclohexanecarbothioamide (0.5 g, 2 mmol) in pyridine (5 ml) at 60 °C was treated with O-benzylhydroxylamine hydrochloride (0.65 g, 4 mmol) and the resulting mixture was then stirred for 24 hours at 60 °C. The reaction mixture was partitioned between water (50 ml) and ethyl acetate (25 ml) and the aqueous layer was then extracted with ethyl acetate (2 x 25ml). The combined organic extracts were washed with brine (50 ml) then dried over magnesium sulphate. Concentration in vacuo gave a crude oil which was recrystallised from ethyl acetate to give (±)-anti-N-methyl-2-benzyloxyimino-1-(3-pyridyl)cyclohexanone (0.57 g, 1.6 mmol), m.p. 128-130 °C. NMR 1.48-2.15 (c, 4H), 2.16-2.40 (m, 2H), 2.8-2.96 (m, 2H), 2.96-3.04 (d, 3H), 5.02-5.08 (s, 2H), 7.12-7.22 (c, 3H), 7.32-7.44 (c, 3H), 7.44-7.56 (m, 2H), 8.16-8.38 (bs, 1H), 8.38-8.52 (c, 2H). Found:- C, 67.7; H, 6.5; N, 11.9; S, 9.0%; calculated:- C, 68.0; H, 6.6; N, 11.9; S, 9.1%.

REFERENCE EXAMPLE 2

By proceeding in a similar manner to that described in Reference Example 1, there was prepared (-)-(S)-anti-2-(2,3,4,5,6,-pentafluorobenzyloxyimino)-N-methyl-1-(3-pyridyl)cyclohexanecarbothioamide, m.p. 115-116 °C, from (S)-N-methyl-2-oxo-1-(3-pyridyl)cyclohexanecarbothioamide.

The present invention includes within its scope pharmaceutical compositions which comtain one or more compounds of general formula (I), or pharmaceutically acceptable salts thereof, in association with a pharmaceutically acceptable carrier or coating. In clinical practice the compounds of the present invention may be administered rectally, but are preferably administered parenterally, by inhalation if appropriate, or, more preferably, orally.

Solid compositions for oral administration include compressed tablets, pills, powders and granules. In such solid compositions, one or more of the active compounds is, or are, admixed with at least one inert diluent such as starch, sucrose or lactose. The compositions may also comprise, as is normal practice, additional substances other than inert diluents, e.g. lubricating agents, such as magnesium stearate.

Liquid compositions for oral administration include pharmaceutically acceptable emulsions, solutions, suspensions, syrups and elixirs containing inert diluents commonly used in the art such as water and liquid paraffin. Besides inert diluents such compositions may comprise adjuvants, such as wetting and suspending agents, and sweetening, flavouring, perfuming and preserving agents. The compositions according to the invention for oral administration also include capsules of absorbable material such as gelatin, containing one or more of the active substances with or without the addition of diluents or excipients.

Preparations according to the invention for parenteral administration include sterile aqueous, aqueous-organic, and organic solutions, suspensions and emulsions. Examples of organic solvents or suspending media are propylene glycol, polyethylene glycol, vegetable oils such as olive oil and injectable organic esters such as ethyl oleate. The compositions may also contain adjuvants such as stabilising, preserving,

wetting, emulsifying and dispersing agents. They may be sterilized by, for example, filtration through a bacteria-retaining filter, by incorporation in the compositions of sterilizing agents, by irradiation or by heating. They may also be manufactured in the form of sterile solid compositions, which can be dissolved in sterile water or some other sterile injectable medium immediately before use.

Compositions for inhalation may be sterile aqueous solutions which are then nebulised or dry powders formulated in accordance with known methods.

Solid compositions for rectal administration include suppositories formulated in accordance with known methods and containing one or more of the compounds of formula (I) or pharmaceutically acceptable salts thereof.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from 0.001 to 50 mg/kg body weight per day by oral administration. By inhalation the preferred daily dosage is from 0.001 to 5 mg/kg body weight.

The compounds may also be applied topically for inhibition of head hair loss associated with male pattern baldness, the preferred daily dosage being from 0.1 to 10 mg/kg body weight applied, for example, in 5ml portions two or three times per day.

The following Composition Example illustrates pharmaceutical compositions according to the present invention.

COMPOSITION EXAMPLE

No. 2 size gelatin capsules each containing:-

| | |
|---|---|
| (±)-trans-2-benzyloxyamino-N-methyl-1-(3-pyridyl)cyclohexanecarbothioamide | 20mg |
| lactose | 100mg |
| starch | 60mg |
| dextrin | 40mg |
| magnesium stearate | 1mg |

were prepared in accordance with the usual procedure.

## Claims

**Claims for the following Contracting States : AT, BE, CH, DE, DK, FR, GB, IT, LI, LU, NL, SE**

**1.** A thioformamide derivative of the general formula:

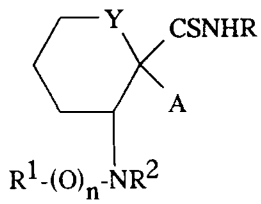

I

wherein R represents a straight- or branched-chain alkyl radical containing from 1 to 4 carbon atoms, A represents an aromatic heterocyclic radical containing one or two nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl and thieno[3,2-b]pyridin-6-yl, or represents a phenyl group substituted in the 3 and/or 5 position with a cyano, nitro, trifluoromethyl, carbamoyl, carboxy, $C_{2-5}$-alkanoyl, $C_{2-5}$-alkoxycarbonyl or $C_{1-4}$-alkylsulphonyl group or a fluorine, chlorine or bromine atom, and optionally further substituted with halogen atom(s), $C_{1-4}$-alkyl or $C_{6-12}$-aryl group(s), or A is a phenyl group unsubstitutued or substituted with halogen atom(s), $C_{1-4}$-alkyl or $C_{6-12}$-aryl group(s) or with substituents which together form a fused ring and Y represents a valency

bond or a methylene or ethylene radical, $R^2$ represents a hydrogen atom, an alkyl, cycloalkyl, cycloalkylalkyl, aryl, aralkyl, aryloxyalkyl, aromatic heterocyclylalkyl or aromatic heterocyclyloxyalkyl group, optionally substituted by one or more halogen atoms, or a group BC(=O)- in which B represents an alkyl, aryl, or aromatic heterocyclic group, optionally substituted by one or more substituents selected from halogen atoms, or B represents a benzyl, phenethyl, 1-naphthylmethyl, 2-naphthylmethyl or pyrid-3-ylmethyl radical, n represents 0 or 1, and when n represents 0 $R^1$ represents a hydrogen atom, an alkyl, cycloalkyl, cycloalkylalkyl, aryl, aralkyl, aryloxyalkyl, aromatic heterocyclylalkyl or aromatic heterocyclyloxyalkyl group, optionally substituted by one or more substituents selected from halogen atoms or a group BC(=O)- or $BSO_2$-, and when n represents 1 $R^1$ represents a straight- or branched-chain alkyl radical containing from 1 to 4 carbon atoms which is unsubstituted or substituted by one or more substituents selected from $C_{2-4}$-alkenyl, carboxy, $C_{2-5}$-alkoxycarbonyl, hydroxy, $C_{1-4}$-alkoxy, carbamoyl (unsubstituted or substituted by one or two $C_{1-4}$-alkyl groups), amino, $C_{1-4}$-alkylamino and di-$C_{1-4}$-alkylamino groups or represents a benzyl, phenethyl, 1-naphthylmethyl, 2-naphthylmethyl or pyrid-3-ylmethyl radical each of which is unsubstituted or substituted on the ring by one or more halogen atoms or hydroxy, $C_{1-4}$-alkyl, $C_{1-4}$-alkoxy (alkoxy being unsubstituted or substituted as defined for alkyl groups represented by $R^1$), cyano, nitro, trifluoromethyl, carboxy, $C_{1-4}$-alkylamino, $C_{2-5}$-alkanoylamino or $C_{2-5}$-alkoxycarbonyl groups, the aryl groups and moities in the definition of $R^1$, $R^2$ and B being selected from phenyl and naphthyl optionally substituted by one or more substituents selected from halogen atoms and the aromatic heterocyclic groups and moities in the definition of $R^1$, $R^2$ and B being selected from pyrid-3-yl and pharmaceutically acceptable salts thereof.

**2.** A compound according to claim 1 wherein Y represents methylene.

**3.** A compound according to claim 1 or 2 wherein A represents 3-pyridyl, 6-chloropyrid-3-yl, 5-bromopyrid-3-yl, 3-quinolinyl, 4-isoquinolinyl or 5-pyrimidyl, or phenyl substituted in the 3 and/or 5 position with an electron-withdrawing group selected from cyano, nitro, trifluoromethyl, carbamoyl, carboxy, $C_{2-5}$-alkanoyl, $C_{2-5}$-alkoxycarbonyl or $C_{1-4}$-alkylsulphonyl group or a fluorine, chlorine or bromine atom and optionally further substituted with halogen atom(s), $C_{1-4}$-alkyl or $C_{6-12}$-aryl group(s), or
the phenyl group A may be substituted with halogen atom(s), $C_{1-4}$alkyl or $C_{6-12}$-aryl group(s) or with substituents which together form a fused ring.

**4.** A compound according to any one of the preceding claims wherein A represents phenyl, 4-chlorophenyl, 3-trifluoromethylphenyl, 3-nitrophenyl, 3,4-dichlorophenyl or 2-naphthyl.

**5.** A compound according to any one of the preceding claims wherein B represents a phenyl or pyrid-3-yl group or a straight- or branched-chain alkyl group (containing from 1 to 6 carbon atoms) each of which being optionally substituted by one or more halogen atoms, or B represents a benzyl, phenethyl, 1-naphthylmethyl, 2-naphthylmethyl or pyrid-3-ylmethyl radical.

**6.** A compound according to any one of the preceding claims wherein B represents a phenyl, 4-fluorophenyl, 3,4-difluorophenyl, pyrid-3-yl, methyl, butyl, 2-benzyl or phenethyl.

**7.** A compound according to any one of the preceding claims 1 to 4 wherein $R^2$ represents a hydrogen atom.

**8.** A compound according to any one of the preceding claims 1 to 4 wherein the amino group $R^1R^2N$- is 1-phenylethylamino, 1-(1-naphthyl)ethylamino, 1-cyclohexylethylamino or 1-(pyrid-3-yl)ethylamino.

**9.** A compound according to claim 1 which comprises one or more of the following features:
(i) R represents methyl;
(ii) A represents 3-pyridyl;
(iii) Y represents methylene;
(iv) B represents lower alkyl or optionally substituted phenyl;
(v) $R^2$ represents hydrogen;
(vi) $R^1$ represents hydrogen, lower alkyl, optionally carrying a substituent selected from napthyl and optionally substituted phenyl or 3-pyridyl, cycloalkyl and hydroxy groups, or represents a lower

alkanoyl or aroyl or arylsulphonyl group.

**10.** A process for the preparation of a thioformamide derivative according to claim 1 or a pharmaceutically acceptable salt thereof which comprises:

(A) when $R^2$ represents the hydrogen atom the reduction of a compound of the general formula:

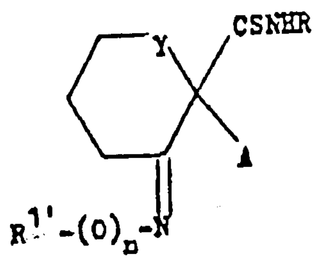

(III)

wherein $R^{1'}$ is as defined in claim 1 for $R^1$ or, when n is 1, $R^{1'}$ may represent the hydrogen atom;

(B) when one or both of $R^1$ and $R^2$ represents an optionally substituted alkyl, cycloalkyl, cycloalkylalkyl, aryl, aralkyl, aryloxyalkyl, aromatic heterocyclylalkyl or aromatic heterocyclyloxyalkyl group by the reaction of a compound of general formula (I) wherein at least one of $R^1$ and $R^2$ represents the hydrogen atom with the corresponding alkyl, cycloalkyl, cycloalkylalkyl, aryl, aralkyl, aryloxyalkyl, aromatic heterocyclylalkyl or aromatic heterocyclyloxyalkyl halide in the absence or presence of an inert organic solvent;

(C) when one or both of $R^1$ and $R^2$ represents a group BC(=O)- as defined in claim 1 by the reaction of a compound of general formula (I) wherein at least one of $R^1$ and $R^2$ represents a hydrogen atom with an acid halide of a compound of general formula:

BCOOH (IV)

or a reactive acid anhydride thereof;

(D) when $R^1$ represents a group $BSO_2$- as defined in claim 1 by the reaction of a compound of general formula (I) wherein n is 0 and $R^1$ represents a hydrogen atom with a sulphonyl halide of the general formula:-

$BSO_2X^1$ (V)

wherein B is as hereinbefore defined and $X^1$ represents a halogen atom;

optionally followed by the conversion of a thioformamide derivative thus obtained into a pharmaceutically acceptable salt thereof.

**11.** A pharmaceutical composition which comprises a thioformamide derivative according to claim 1 or a pharmaceutically acceptable salt thereof in association with a pharmaceutically acceptable carrier or coating.

**12.** A thioformamide derivative according to claim 1 or a pharmaceutically acceptable salt thereof for use as a medicament.

**Claims for the following Contracting States : ES, GR**

**1.** A process for the preparation of a thioformamide derivative of the general formula:

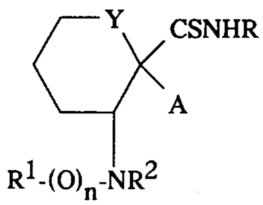

I

wherein R represents a straight- or branched-chain alkyl radical containing from 1 to 4 carbon atoms, A represents an aromatic heterocyclic radical containing one or two nitrogen atoms (optionally substituted by a straight- or branched-chain alkyl or alkoxy radical containing 1 to 4 carbon atoms or by a halogen atom), selected from pyrid-3-yl, isoquinolin-4-yl, tetrahydroquinolin-3-yl, quinolin-3-yl, pyridazin-4-yl, pyrimid-5-yl, thiazol-5-yl, thieno[2,3-b]pyridin-5-yl, pyrazin-2-yl, indol-3-yl and thieno[3,2-b]pyridin-6-yl, or represents a phenyl group substituted in the 3 and/or 5 position with a cyano, nitro, trifluoromethyl, carbamoyl, carboxy, $C_{2-5}$-alkanoyl, $C_{2-5}$-alkoxycarbonyl or $C_{1-4}$-alkylsulphonyl group or a fluorine, chlorine or bromine atom, and optionally further substituted with halogen atom(s), $C_{1-4}$-alkyl or $C_{6-12}$-aryl group(s), or A is a phenyl group unsubstituted or substituted with halogen atom(s), $C_{1-4}$-alkyl or $C_{6-12}$-aryl group(s) or with substituents which together form a fused ring and Y represents a valency bond or a methylene or ethylene radical, $R^2$ represents a hydrogen atom, an alkyl, cycloalkyl, cycloalkylalkyl, aryl, aralkyl, aryloxyalkyl, aromatic heterocyclylalkyl or aromatic heterocyclyloxyalkyl group, optionally substituted by one or more substituents selected from halogen atoms or a group BC(=O)- in which B represents an alkyl, aryl, or aromatic heterocyclic group, optionally substituted by one or more halogen atoms or B represents a benzyl, phenethyl, 1-naphthylmethyl, 2-naphthylmethyl or pyrid-3-ylmethyl radical, n represents 0 or 1, and when n represents 0 $R^1$ represents a hydrogen atom, an alkyl, cycloalkyl, cycloalkylalkyl, aryl, aralkyl, aryloxyalkyl, aromatic heterocyclylalkyl or aromatic heterocyclyloxyalkyl group, optionally substituted by one or more halogen atoms or a group BC(=O)- or $BSO_2$-, ad when n represents 1 $R^1$ represents a straight- or branched-chain alkyl radical containing from 1 to 4 carbon atoms which is unsubstituted or substituted by one or more substituents selected from $C_{2-4}$-alkenyl, carboxy, $C_{2-5}$-alkoxycarbonyl, hydroxy, $C_{1-4}$-alkoxy, carbamoyl (unsubstituted or substituted by one or two $C_{1-4}$-alkyl groups), amino, $C_{1-4}$-alkylamino and di-$C_{1-4}$-alkylamino groups or represents a benzyl, phenethyl, 1-naphthylmethyl, 2-naphthylmethyl or pyrid-3-ylmethyl radical each of which is unsubstituted or substituted on the ring by one or more halogen atoms or hydroxy, $C_{1-4}$alkyl, $C_{1-4}$-alkoxy (alkoxy being unsubstituted or substituted as defined for alkyl groups represented by $R^1$), cyano, nitro, trifluoromethyl, carboxy, $C_{1-4}$-alkylamino, $C_{2-5}$-alkanoylamino or $C_{2-5}$-alkoxycarbonyl groups, the aryl groups and moities in the definition of $R^1$, $R^2$ and B being selected from phenyl and naphthyl optionally substituted by one or more substituents selected from halogen atoms and the aromatic heterocyclic groups and moities in the definition of $R^1$, $R^2$ and B being selected from pyrid-3-yl and pharmaceutically acceptable salts thereof, which process comprises:

(A) when $R^2$ represents the hydrogen atom the reduction of a compound of the general formula:

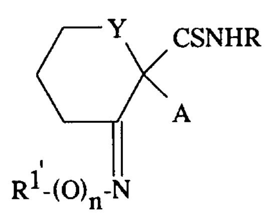

III

wherein $R^{1'}$ is as hereinbefore defined for $R^1$ or, when n is 1, $R^1$ may represent a hydrogen atom;

(B) when one or both of $R^1$ and $R^2$ represents an optionally substituted alkyl, cycloalkyl, cycloalkylalkyl, aryl, aralkyl, aryloxyalkyl, aromatic heterocyclylalkyl or aromatic heterocyclyloxyalkyl group by the reaction of a compound of general formula (I) wherein at least one of $R^1$ and $R^2$ represents the

hydrogen atom with the corresponding alkyl, cycloalkyl, cycloalkylalkyl, aryl, aralkyl, aryloxyalkyl, aromatic heterocyclylalkyl or aromatic heterocyclyloxyalkyl halide in the absence or presence of an inert organic solvent;

(C) when one or both of $R^1$ and $R^2$ represents a group BC(=O)- as hereinbefore defined by the reaction of a compound of general formula (I) wherein at least one of $R^1$ and $R^2$ represents a hydrogen atom with an acid halide of a compound of general formula:

BCOOH (IV)

or a reactive acid anhydride thereof;

(D) when $R^1$ represents a group $BSO_2$- as hereinbefore defined by the reaction of a compound of general formula (I) wherein n is 0 and $R^1$ represents a hydrogen atom with a sulphonyl halide of the general formula:-

$BSO_2X^1$ (V)

wherein B is as hereinbefore defined and $X^1$ represents a halogen atom;

optionally followed by the conversion of a thioformamide derivative thus obtained into a pharmaceutically acceptable salt thereof.

**2.** A process according to claim 1 wherein Y represents methylene.

**3.** A process according to claim 1 or 2 wherein A represents 3-pyridyl, 6-chloropyrid-3-yl, 5-bromopyrid-3-yl, 3-quinolinyl, 4-isoquinolinyl or 5-pyrimidyl, or phenyl substituted in the 3 and/or 5 position with an electron-withdrawing group selected from cyano, nitro, trifluoromethyl, carbamoyl, carboxy, $C_{2-5}$-alkanoyl, $C_{2-5}$-alkoxycarbonyl or $C_{1-4}$-alkylsulphonyl group or a fluorine, chlorine or bromine atom and optionally further substituted with halogen atom(s), $C_{1-4}$-alkyl or $C_{6-12}$-aryl group(s), or
the phenyl group A may be substituted with halogen atom(s), $C_{1-4}$alkyl or $C_{6-12}$-aryl group(s) or with substituents which together form a fused ring.

**4.** A process according to any one of the preceding claims wherein A represents phenyl, 4-chlorophenyl, 3-trifluoromethylphenyl, 3-nitrophenyl, 3,4-dichlorophenyl or 2-naphthyl.

**5.** A process according to any one of the preceding claims wherein B represents a phenyl or pyrid-3-yl group or a straight- or branched-chain alkyl group (containing from 1 to 6 carbon atoms) each of which being optionally substituted by one or more halogen atoms, or B represents a benzyl, phenethyl, 1-naphthylmethyl, 2-naphthylmethyl or pyrid-3-ylmethyl radical.

**6.** A process according to any one of the preceding claims wherein B represents a phenyl, 4-fluorophenyl, 3,4-difluorophenyl, pyrid-3-yl, methyl, butyl, benzyl or phenethyl.

**7.** A process according to any one of the preceding claims 1 to 4 wherein $R^2$ represents a hydrogen atom.

**8.** A process according to any one of the preceding claims 1 to 4 wherein the amino group $R^1R^2N$- is 1-phenylethylamino, 1-(1-naphthyl)ethylamino, 1-cyclohexylethylamino or 1-(pyrid-3-yl)ethylamino.

**9.** A process according to claim 1 which comprises one or more of the following features:

(i) R represents methyl;

(ii) A represents 3-pyridyl;

(iii) Y represents methylene;

(iv) B represents lower alkyl or optionally substituted phenyl;

(v) $R^2$ represents hydrogen;

(vi) $R^1$ represents hydrogen, lower alkyl, optionally carrying a substituent selected from napthyl and optionally substituted phenyl or 3-pyridyl, cycloalkyl and hydroxy groups, or represents a lover alkanoyl or aroyl or arylsulphonyl group.

**Patentansprüche**
**Patentansprüche für folgende Vertragsstaaten : AT, BE, CH, DE, DK, FR, GB, IT, LI, LU, NL, SE**

1. Thioformamidderivat der allgemeinen Formel

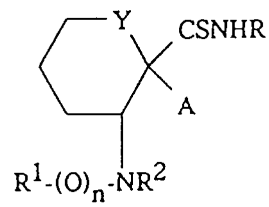

I

worin R für einen gerad- oder verzweigtkettigen Alkylrest steht, der 1 bis 4 Kohlenstoffatome enthält, A für einen aromatischen heterocyclischen Rest steht, der ein oder zwei Stickstoffatome enthält (wahlweise substituiert durch einen gerad- oder verzweigtkettigen Alkyl- oder Alkoxyrest, der 1 bis 4 Kohlenstoffatome enthält, oder durch ein Halogenatom), ausgewählt aus Pyrid-3-yl, Isochinolin-4-yl, Tetrahydrochinolin-3-yl, Chinolin-3-yl, Pyridazin-4-yl, Pyrimid-5-yl, Thiazol-5-yl, Thieno[2,3-b]pyridin-5-yl, Pyrazin-2-yl, Indol-3-yl und Thieno[3,2-b]pyridin-6-yl, oder für eine Phenylgruppe steht, die an der Position 3 und/oder 5 substituiert ist mit einer Cyano-, Nitro-, Trifluormethyl-, Carbamoyl-, Carboxy-, $C_{2-5}$-Alkanoyl-, $C_{2-5}$-Alkoxycarbonyl- oder $C_{1-4}$-Alkylsulfonylgruppe oder einem Fluor-, Chlor- oder Bromatom, und ferner wahlweise substituiert ist mit Halogenatom(en), $C_{1-4}$-Alkyl- oder $C_{6-12}$-Arylgruppe(n), oder A eine Phenylgruppe ist, die unsubstituiert oder substituiert ist mit Halogenatom(en), $C_{1-4}$-Alkyl- oder $C_{6-12}$-Arylgruppe(n) oder mit Substituenten, die zusammen einen fusionierten Ring bilden, und Y für eine Valenzbindung oder einen Methylen- oder Ethylenrest steht, $R^2$ für ein Wasserstoffatom, eine Alkyl-, Cycloalkyl-, Cycloalkylalkyl-, Aryl-, Aralkyl-, Aryloxyalkyl-, aromatische Heterocyclylalkyl- oder aromatische Heterocyclyloxyalkylgruppe, wahlweise substituiert durch ein oder mehrere Halogenatome, oder eine Gruppe BC(=O)- steht, in der B für eine Alkyl-, Aryl- oder aromatische heterocyclische Gruppe steht, wahlweise durch einen oder mehrere Substituenten substituiert, ausgewählt aus Halogenatomen, oder B für einen Benzyl-, Phenethyl-, 1-Naphthylmethyl-, 2-Naphthylmethyl- oder Pyrid-3-yl-methylrest steht, n für 0 oder 1 steht, und wenn n für 0 steht, dann steht $R^1$ für ein Wasserstoffatom, eine Alkyl-, Cycloalkyl-, Cycloalkylalkyl-, Aryl-, Aralkyl-, Aryloxyalkyl-, aromatische Heterocyclylalkyl- oder aromatische Heterocyclyloxyalkylgruppe, wahlweise substituiert durch einen oder mehrere Substituenten, ausgewählt aus Halogenatomen, oder eine Gruppe BC(=O)- oder $BSO_2$-, und wenn n für 1 steht, dann steht $R^1$ für einen gerad- oder verzweigtkettigen Alkylrest, der 1 bis 4 Kohlenstoffatome enthält, welcher unsubstituiert ist oder durch einen oder mehrere Substituenten substituiert ist, ausgewählt aus $C_{2-4}$-Alkenyl-, Carboxy-, $C_{2-5}$-Alkoxycarbonyl-, Hydroxy-, $C_{1-4}$-Alkoxy-, Carbamoyl(unsubstituiert oder durch ein oder zwei $C_{1-4}$-Alkylgruppen substituiert), Amino-, $C_{1-4}$-Alkylamino- und Di-$C_{1-4}$-Alkylaminogruppen oder für einen Benzyl-, Phenetyl-, 1-Naphthylmethyl-, 2-Naphthylmethyl- oder Pyrid-3-ylmethylrest steht, von denen jeder unsubstituiert oder am Ring substituiert ist durch ein oder mehrere Halogenatome oder Hydroxy-, $C_{1-4}$-Alkyl-, $C_{1-4}$-Alkoxy (unsubstituiertes oder substituiertes Alkoxy, wie dies für die Alkylgruppen definiert wurde, für die $R^1$ steht), Cyano-, Nitro-, Trifluormethyl-, Carboxy-, $C_{1-4}$-Alkylamino-, $C_{2-5}$-Alkanoylamino- oder $C_{2-5}$-Alkoxycarbonylgruppen, die Arylgruppen und Reste der Definition von $R^1$, $R^2$ und B ausgewählt werden aus Phenyl und Naphthyl, wahlweise durch einen oder mehrere Substituenten substituiert, ausgewählt aus Halogenatomen, und die aromatischen heterocyclischen Gruppen und Reste der Definition von $R^1$, $R^2$ und B ausgewählt werden aus Pyrid-3-yl und pharmazeutisch annehmbaren Salzen hiervon.

2. Verbindung nach Anspruch 1, worin Y für Methylen steht.

3. Verbindung nach Anspruch 1 oder 2, worin A steht für 3-Pyridyl-, 6-Chlorpyrid-3-yl, 5-Brompyrid-3-yl, 3-Chinolinyl, 4-Isochinolinyl oder 5-Pyrimidyl oder Phenyl, das an der Position 3 und/oder 5 substituiert ist mit einer elektronenziehenden Gruppe, ausgewählt aus einer Cyano-, Nitro-, Trifluormethyl-, Carbamoyl-, Carboxy-, $C_{2-5}$-Alkanoyl-, $C_{2-5}$-Alkoxycarbonyl- oder $C_{1-4}$-Alkylsulfonylgruppe oder einem Fluor-, Chlor- oder Bromatom, und ferner wahlweise substituiert ist mit Halogenatom(en), $C_{1-4}$-Alkyl- oder $C_{6-12}$-Arylgruppe(n), oder die Phenylgruppe A substituiert sein kann mit Halogenatom(en), $C_{1-4}$-Alkyl- oder $C_{6-12}$-Arylgruppe(n) oder mit Substituenten, die zusammen einen fusionierten Ring bilden.

4. Verbindung nach einem der vorangehenden Ansprüche, worin A für Phenyl, 4-Chlorphenyl, 3-Trifluormethylphenyl, 3-Nitrophenyl, 3,4-Dichlorphenyl oder 2-Naphthyl steht.

5. Verbindung nach einem der vorangehenden Ansprüche, worin B für eine Phenyl- oder Pyrid-3-ylgruppe oder eine gerad- oder verzweigtkettige Alkylgruppe (die 1 bis 6 Kohlenstoffatome enthält) steht, wobei hiervon jede wahlweise durch ein oder mehrere Halogenatome substituiert sein kann, oder B für einen Benzyl-, Phenethyl-, 1-Naphthylmethyl-, 2-Naphthylmethyl- oder Pyrid-3-ylmethylrest steht.

6. Verbindung nach einem der vorangehenden Ansprüche, worin B für Phenyl , 4-Fluorphenyl , 3,4-Difluorphenyl, Pyrid-3-yl, Methyl, Butyl, Benzyl oder Phenethyl steht.

7. Verbindung nach einem der vorangehenden Ansprüche 1 bis 4, worin $R^2$ für ein Wasserstoffatom steht.

8. Verbindung nach einem der vorangehenden Ansprüche 1 bis 4, worin die Aminogruppe $R^1R^2N$- für 1-Phenylethylamino, 1-(1-Naphthyl)ethylamino, 1-Cyclohexylethylamino oder 1-(Pyrid-3-yl)ethylamino steht.

9. Verbindung nach Anspruch 1, die eines oder mehrere der folgenden Merkmale umfaßt:
(i) R steht für Methyl,
(ii) A steht für 3-Pyridyl,
(iii) Y steht für Methylen,
(iv) B steht für ein niederes Alkyl oder wahlweise substituiertes Phenyl,
(v) $R^2$ steht für Wasserstoff,
(vi) $R^1$ steht für Wasserstoff, niederes Alkyl, das wahlweise einen Substituenten trägt, ausgewählt aus Naphthyl und wahlweise substituiertem Phenyl oder 3-Pyridyl-, Cycloalkyl- und Hydroxygruppen, oder für eine niedere Alkanoyl- oder Aroyl- oder Arylsulfonylgruppe steht.

10. Verfahren zur Herstellung eines Thioformamidderivats nach Anspruch 1 oder eines pharmazeutisch annehmbaren Salzes hiervon, gekennzeichnet durch:
(A) wenn $R^2$ für das Wasserstoffatom steht, die Reduktion einer Verbindung der allgemeinen Formel:

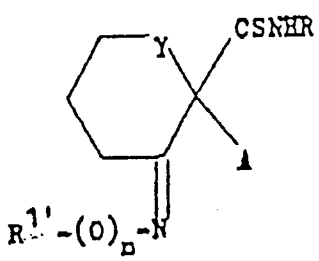

(III)

worin $R^{1'}$ wie $R^1$ nach Anspruch 1 definiert ist, oder wenn n für 1 steht kann $R^{1'}$ für das Wasserstoffatom stehen,
(B) wenn eines oder beide von $R^1$ und $R^2$ für eine wahlweise substituierte Alkyl-, Cycloalkyl-, Cycloalkylalkyl-, Aryl-, Aralkyl-, Aryloxyalkyl-, aromatische Heterocyclylalkyl- oder aromatische Heterocyclyloxyalkylgruppe stehen, durch die Umsetzung einer Verbindung der allgemeinen Formel (I), worin zumindest eines von $R^1$ und $R^2$ für das Wasserstoffatom steht, mit dem entsprechenden Alkyl-, Cycloalkyl-, Cycloalkylalkyl-, Aryl-, Aralkyl-, Aryloxyalkyl-, aromatischen Heterocyclylalkyl- oder aromatischem Heterocyclyloxyalkylhalogenid in Abwesenheit oder Gegenwart eines inerten organischen Lösungsmittels,
(C) wenn eines oder beide von $R^1$ und $R^2$ für eine Gruppe BC(=O)- nach Anspruch 1 stehen, durch die Umsetzung einer Verbindung der allgemeinen Formel (I), worin zumindest eines von $R^1$ und $R^2$ für ein Wasserstoffatom steht, mit einem Säurehalogenid einer Verbindung der allgemeinen Formel:

BCOOH (IV)

oder einem reaktiven Säureanhydrid hiervon,

(D) wenn $R^1$ für eine Gruppe $BSO_2$- nach Anspruch 1 steht, durch die Umsetzung einer Verbindung der allgemeinen Formel (I), worin n für 0 steht und $R^1$ für ein Wasserstoffatom steht, mit einem Sulfonylhalogenid der allgemeinen Formel:

$BSO_2X^1$ (V)

worin B wie hierin vorher definiert ist und $X^1$ für ein Halogenatom steht, wahlweise gefolgt von der Umwandlung eines so erhaltenen Thioformamidderivats in ein pharmazeutisch annehmbares Salz hiervon.

**11.** Pharmazeutische Zusammensetzung, die ein Thioformamidderivat nach Anspruch 1 oder ein pharmazeutisch annehmbares Salz hiervon zusammen mit einem pharmazeutisch annehmbaren Träger oder einer pharmazeutisch annehmbaren Beschichtung umfaßt.

**12.** Thioformamidderivat nach Anspruch 1 oder ein pharmazeutisch annehmbares Salz hiervon zur Verwendung als Medikament.

**Patentansprüche für folgende Vertragsstaaten : ES, GR**

**1.** Verfahren zur Herstellung eines Thioformamidderivats der allgemeinen Formel

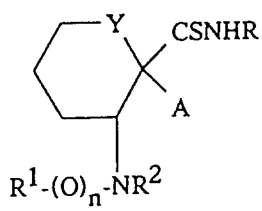

I

worin R für einen gerad- oder verzweigtkettigen Alkylrest steht, der 1 bis 4 Kohlenstoffatome enthält, A für einen aromatischen heterocyclischen Rest steht, der ein oder zwei Stickstoffatome enthält (wahlweise substituiert durch einen gerad- oder verzweigtkettigen Alkyl- oder Alkoxyrest, der 1 bis 4 Kohlenstoffatome enthält, oder durch ein Halogenatom), ausgewählt aus Pyrid-3-yl, Isochinolin-4-yl, Tetrahydrochinolin-3-yl, Chinolin-3-yl, Pyridazin-4-yl, Pyrimid-5-yl, Thiazol-5-yl, Thieno[2,3-b]pyridin-5-yl, Pyrazin-2-yl, Indol-3-yl und Thieno[3,2-b]pyridin-6-yl, oder für eine Phenylgruppe steht, die an der Position 3 und/oder 5 substituiert ist mit einer Cyano-, Nitro-, Trifluormethyl-, Carbamoyl-, Carboxy-, $C_{2-5}$-Alkanoyl-, $C_{2-5}$-Alkoxycarbonyl- oder $C_{1-4}$-Alkylsulfonylgruppe oder einem Fluor-, Chlor- oder Bromatom, und ferner wahlweise substituiert ist mit Halogenatom(en), $C_{1-4}$-Alkyl- oder $C_{6-12}$-Arylgruppe(n), oder A eine Phenylgruppe ist, die unsubstituiert oder substituiert ist mit Halogenatom(en), $C_{1-4}$-Alkyl- oder $C_{6-12}$-Arylgruppe(n) oder mit Substituenten, die zusammen einen fusionierten Ring bilden, und Y für eine Valenzbindung oder einen Methylen- oder Ethylenrest steht, $R^2$ für ein Wasserstoffatom, eine Alkyl-, Cycloalkyl-, Cycloalkylalkyl-, Aryl-, Aralkyl-, Aryloxyalkyl-, aromatische Heterocyclylalkyl- oder aromatische Heterocyclyloxyalkylgruppe, wahlweise substituiert durch einen oder mehrere Substituenten, ausgewählt aus Halogenatomen, oder eine Gruppe BC(=O)- steht, in der B für eine Alkyl-, Aryl- oder aromatische heterocyclische Gruppe steht, wahlweise substituiert durch ein oder mehrere Halogenatome, oder B für einen Benzyl-, Phenethyl-, 1-Naphthylmethyl-, 2-Naphthylmethyl- oder Pyrid-3-yl-methylrest steht, n für 0 oder 1 steht, und wenn n für 0 steht, dann steht $R^1$ für ein Wasserstoffatom, eine Alkyl-, Cycloalkyl-, Cycloalkylalkyl-, Aryl-, Aralkyl-, Aryloxyalkyl-, aromatische Heterocyclylalkyl- oder aromatische Heterocyclyloxyalkylgruppe, wahlweise substituiert durch ein oder mehrere Halogenatome, oder eine Gruppe BC(=O)- oder $BSO_2$-, und wenn n für 1 steht, dann steht $R^1$ für einen gerad- oder verzweigtkettigen Alkylrest, der 1 bis 4 Kohlenstoffatome enthält, welcher unsubstituiert ist oder durch einen oder mehrere Substituenten substituiert ist, ausgewählt aus $C_{2-4}$-Alkenyl-, Carboxy-, $C_{2-5}$-Alkoxycarbonyl-, Hydroxy-, $C_{1-4}$-Alkoxy-, Carbamoyl- (unsubstituiert oder durch ein oder zwei $C_{1-4}$-Alkylgruppen substituiert), Amino-, $C_{1-4}$-Alkylamino- und Di-$C_{1-4}$-Alkylaminogruppen oder für einen Benzyl-, Phenetyl-, 1-Naphthylmethyl-, 2-Naphthylmethyl- oder Pyrid-3-ylmethylrest steht, von denen jeder unsubstituiert oder am Ring substituiert ist durch ein oder mehrere

Halogenatome oder Hydroxy-, $C_{1-4}$-Alkyl-, $C_{1-4}$-Alkoxy (unsubstituiertes oder substituiertes Alkoxy, wie dies für die Alkylgruppen definiert wurde, für die $R^1$ steht), Cyano-, Nitro-, Trifluormethyl-, Carboxy-, $C_{1-4}$-Alkylamino-, $C_{2-5}$-Alkanoylamino- oder $C_{2-5}$-Alkoxycarbonylgruppen, die Arylgruppen und Reste der Definition von $R^1$, $R^2$ und B ausgewählt werden aus Phenyl und Naphthyl, wahlweise durch einen oder mehrere Substituenten substituiert, ausgewählt aus Halogenatomen, und die aromatischen heterocyclischen Gruppen und Reste der Definition von $R^1$, $R^2$ und B, ausgewählt werden aus Pyrid-3-yl und pharmazeutisch annehmbaren Salzen hiervon, wobei das Verfahren gekennzeichnet ist durch

(A) wenn $R^2$ für das Wasserstoffatom steht, die Reduktion einer Verbindung der allgemeinen Formel:

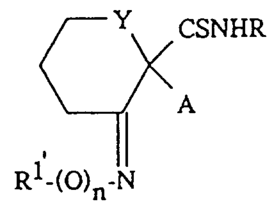

III

worin $R^{1'}$ wie $R^1$ nach Anspruch 1 definiert ist, oder wenn n für 1 steht kann $R^{1'}$ für ein Wasserstoffatom stehen,

(B) wenn eines oder beide von $R^1$ und $R^2$ für eine wahlweise substituierte Alkyl-, Cycloalkyl-, Cycloalkylalkyl-, Aryl-, Aralkyl-, Aryloxyalkyl-, aromatische Heterocyclylalkyl- oder aromatische Heterocyclyloxyalkylgruppe stehen, durch die Umsetzung einer Verbindung der allgemeinen Formel (I), worin zumindest eines von $R^1$ und $R^2$ für das Wasserstoffatom steht, mit dem entsprechenden Alkyl-, Cycloalkyl-, Cycloalkylalkyl-, Aryl-, Aralkyl-, Aryloxyalkyl-, aromatischen Heterocyclylalkyl- oder aromatischen Heterocyclyloxyalkylhalogenid in Abwesenheit oder Gegenwart eines inerten organischen Lösungsmittels,

(C) wenn eines oder beide von $R^1$ und $R^2$ für eine Gruppe BC(=O)- wie hierin vorher definiert stehen, durch die Umsetzung einer Verbindung der allgemeinen Formel (I), worin zumindest eines von $R^1$ und $R^2$ für ein Wasserstoffatom steht, mit einem Säurehalogenid einer Verbindung der allgemeinen Formel:

BCOOH (IV)

oder einem reaktiven Säureanhydrid hiervon,

(D) wenn $R^1$ für eine Gruppe $BSO_2$- wie hierin vorher definiert steht, durch die Umsetzung einer Verbindung der allgemeinen Formel (I), worin n für 0 steht und $R^1$ für ein Wasserstoffatom steht, mit einem Sulfonylhalogenid der allgemeinen Formel:

$BSO_2X^1$ (V)

worin B wie hierin vorher definiert ist und $X^1$ für ein Halogenatom steht, wahlweise gefolgt von der Umwandlung eines so erhaltenen Thioformamidderivats in ein pharmazeutisch annehmbares Salz hiervon.

**2.** Verfahren nach Anspruch 1, worin Y für Methylen steht.

**3.** Verfahren nach Anspruch 1 oder 2, worin A steht für 3-Pyridyl-, 6-Chlorpyrid-3-yl, 5-Brompyrid-3-yl, 3-Chinolinyl, 4-Isochinolinyl oder 5-Pyrimidyl oder Phenyl, das an der Position 3 und/oder 5 substituiert ist mit einer elektronenziehenden Gruppe, ausgewählt aus einer Cyano-, Nitro-, Trifluormethyl-, Carbamoyl-, Carboxy-, $C_{2-5}$-Alkanoyl-, $C_{2-5}$-Alkoxycarbonyl- oder $C_{1-4}$-Alkylsulfonylgruppe oder einem Fluor-, Chlor- oder Bromatom, und ferner wahlweise substituiert ist mit Halogenatom(en), $C_{1-4}$-Alkyl- oder $C_{6-12}$-Arylgruppe(n), oder die Phenylgruppe A substituiert sein kann mit Halogenatom(en), $C_{1-4}$-Alkyl- oder $C_{6-12}$-Arylgruppe(n) oder mit Substituenten, die zusammen einen fusionierten Ring bilden.

**4.** Verfahren nach einem der vorangehenden Ansprüche, worin A für Phenyl, 4-Chlorphenyl, 3-Trifluormethylphenyl, 3-Nitrophenyl, 3,4-Dichlorphenyl oder 2-Naphthyl steht.

**5.** Verfahren nach einem der vorangehenden Ansprüche, worin B für eine Phenyl- oder Pyrid-3-yl-gruppe oder eine gerad- oder verzweigtkettige Alkylgruppe (die 1 bis 6 Kohlenstoffatome enthält) steht, wobei hiervon jede wahlweise durch ein oder mehrere Halogenatome substituiert sein kann, oder B für einen Benzyl-, Phenethyl-, 1-Naphthylmethyl-, 2-Naphthylmethyl- oder Pyrid-3-ylmethylrest steht.

**6.** Verfahren nach einem der vorangehenden Ansprüche, worin B für Phenyl , 4-Fluorphenyl , 3,4-Difluorphenyl, Pyrid-3-yl, Methyl, Butyl, Benzyl oder Phenethyl steht.

**7.** Verfahren nach einem der vorangehenden Ansprüche 1 bis 4, worin $R^2$ für ein Wasserstoffatom steht.

**8.** Verfahren nach einem der vorangehenden Ansprüche 1 bis 4, worin die Aminogruppe $R^1R^2N$- für 1-Phenylethylamino, 1-(1-Naphthyl)ethylamino, 1-Cyclohexylethylamino oder 1-(Pyrid-3-yl)ethylamino steht.

**9.** Verfahren nach Anspruch 1, das eines oder mehrere der folgenden Merkmale umfaßt:
(i) R steht für Methyl,
(ii) A steht für 3-Pyridyl,
(iii) Y steht für Methylen,
(iv) B steht für ein niederes Alkyl oder wahlweise substituiertes Phenyl,
(v) $R^2$ steht für Wasserstoff,
(vi) $R^1$ steht für Wasserstoff, niederes Alkyl, das wahlweise einen Substituenten trägt, ausgewählt aus Naphthyl und wahlweise substituiertem Phenyl oder 3-Pyridyl-, Cycloalkyl- und Hydroxygruppen, oder für eine niedere Alkanoyl- oder Aroyl- oder Arylsulfonylgruppe steht.

**Revendications**
**Revendications pour les Etats contractants suivants : AT, BE, CH, DE, DK, FR, GB, IT, LI, LU, NL, SE**

**1.** Dérivé de thioformamide de formule générale:

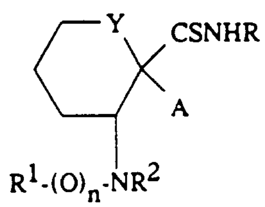

I

dans laquelle R représente un radical alkyle à chaîne droite ou ramifiée contenant de 1 à 4 atomes de carbone, A représente un radical hétérocyclique aromatique contenant un ou deux atomes d'azote (éventuellement substitués par un radical alkyle ou alcoxy à chaîne droite ou ramifiée contenant 1 à 4 atomes de carbone, ou par un atome d'halogène), choisi parmi le pyride-3-yle, l'isoquinoléine-4-yle, le tétrahydroquinoléine-3-yle, le quinoléine-3-yle, le pyridazine-4-yle, le pyrimide-5-yle, le thiazole-5-yle, le thiéno[2,3-b]pyridine-5-yle, le pyrazine-2-yle, l'indole-3-yle et le thiéno[2,3-b]pyridine-6-yle, ou représente un groupe phényle substitué en position 3 et/ou 5 par un groupe cyano, nitro, trifluorométhyle, carbamoyle, carboxy, alcanoyle en $C_2$-$C_5$,alcoxycarbonyle en $C_2$-$C_5$, alkylsulfonyle en $C_1$-$C_4$, ou un atome de fluor, de chlore ou de brome et, éventuellement, encore substitué par un ou des atome(s) d'halogène, un ou des groupe(s) alkyle en $C_1$-$C_4$ ou aryle en $C_6$-$C_{12}$, ou A est un groupe phényle non substitué ou substitué par un ou des atome(s) d'halogène, un ou des groupe(s) alkyle en $C_1$-$C_4$ ou aryle en $C_6$-$C_{12}$ ou par des substituants qui forment ensemble un noyau condensé, et Y représente une liaison de valence ou un radical méthylène ou éthylène, $R^2$ représente un atome d'halogène, un groupe alkyle, cycloalkyle, cycloalkylalkyle, aryle, arylalkyle, aryloxyalkyle, hétérocyclylalkyle aromatique ou hétérocyclyloxyalkyle aromatique, éventuellement substitué par un ou plusieurs atomes d'halogène, ou un groupe BC(=O)- dans lequel B représente un groupe alkyle, aryle ou un groupe hétérocyclique aromatique, éventuellement substitué par un ou plusieurs substituants choisis parmi les atomes d'halogène, ou B représente un radical benzyle, phénéthyle, 1-naphtylméthyle, 2-naphtylméthyle ou pyride-3-yl-méthyle, n représente 0 ou 1, et, lorsque n représente 0, $R^1$ représente un atome d'hydrogène, un groupe alkyle, cycloalkyle, cycloalkylalkyle, aryle, arylalkyle, aryloxyalkyle, hétérocyclylalkyle aromatique ou hétérocyclyloxyalkyle aromatique, éventuellement substitué par un ou plusieurs substituants choisis parmi les atomes d'halogène ou un groupe BC(=O)- ou $BSO_2$-, et, lorsque n représente 1, $R^1$ représente un radical alkyle à chaîne droite ou ramifiée contenant de 1 à 4 atomes de carbone qui est non substitué ou substitué par un ou plusieurs substituants choisis parmi les groupes alcényle en $C_2$-$C_4$, carboxy, alcoxycarbonyle en $C_2$-$C_5$, hydroxy, alcoxy en $C_1$-$C_4$, carbamoyle (non substitué ou substitué par un ou deux groupes alkyle en $C_1$-$C_4$), amino, alkylamino en $C_1$-$C_4$ et di-alkylamino en $C_1$-$C_4$, ou représente un groupe benzyle, phénéthyle, 1-naphtylméthyle, 2-naphtylméthyle ou pyride-3-ylméthyle, chacun d'entre eux étant non substitué ou substitué sur le noyau par un ou plusieurs atomes d'halogène ou par les groupes hydroxy, alkyle en $C_1$-$C_4$, alcoxy en $C_1$-$C_4$ (le groupe alcoxy étant non substitué ou substitué comme défini pour les groupes alkyle représentés par $R^1$), cyano, nitro, trifluorométhyle, carboxy, alkylamino en $C_1$-$C_4$, alcanoylamino en $C_2$-$C_5$ ou alcoxycarbonyle en $C_2$-$C_5$, les groupes et les groupements aryle, dans la définition de $R^1$, $R^2$ et B, étant choisis parmi les groupes phényle et naphtyle, éventuellement substitués par un ou plusieurs substituants choisis parmi les atomes d'halogène, et les groupes et les groupements hétérocycliques aromatiques, dans la définition de $R^1$, $R^2$ et B, étant choisis parmi le groupe pyride-3-yle, et les sels pharmaceutiquement acceptables de ce composé.

**2.** Composé selon la revendication 1, dans lequel Y représente le groupe méthylène.

**3.** Composé selon la revendication 1 ou 2, dans lequel A représente un groupe 3-pyridyle, 6-chloropyride-3-yle, 5-bromopyride-3-yle, 3-quinoléinyle, 4-isoquinoléinyle ou 5-pyrimidyle, ou un groupe phényle substitué en position 3 et/ou 5 par un groupe attracteur d'électrons choisi parmi les groupes cyano, nitro, trifluorométhyle, carbamoyle, carboxy, alcanoyle en $C_2$-$C_5$, alcoxycarbonyle en $C_2$-$C_5$ ou alkylsulfonyle en $C_1$-$C_4$, ou un atome de fluor, de chlore ou de brome et, éventuellement, encore substitué par un ou des atome(s) d'halogène, un ou des groupe(s) alkyle en $C_1$-$C_4$ ou aryle en $C_6$-$C_{12}$, ou le groupe phényle A peut être substitué par un ou des atome(s) d'halogène, un ou des groupe(s) alkyle en $C_1$-$C_4$ ou aryle en $C_6$-$C_{12}$, ou par des substituants qui forment ensemble un noyau condensé.

**4.** Composé selon l'une quelconque des revendications précédentes, dans lequel A représente un groupe phényle, 4-chlorophényle, 3-trifluorométhylphényle, 3-nitrophényle, 3,4-dichlorophényle ou 2-naphtyle.

**5.** Composé selon l'une quelconque des revendications précédentes dans lequel B représente un groupe phényle ou pyride-3-yle ou un groupe alkyle à chaîne droite ou ramifiée (contenant de 1 à 6 atomes de carbone), chacun d'entre eux étant éventuellement substitué par un ou plusieurs atomes d'halogène, ou B représente un radical benzyle, phénéthyle, 1-naphtylméthyle, 2-naphtylméthyle ou pyride-3-ylméthyle.

**6.** Composé selon l'une quelconque des revendications précédentes, dans lequel B représente un groupe phényle, 4-fluorophényle, 3,4-difluorophényle, pyride-3-yle, méthyle, butyle, benzyle ou phénéthyle.

**7.** Composé selon l'une quelconque des revendications 1 à 4 précédentes, dans lequel $R^2$ représente un atome d'hydrogène.

**8.** Composé selon l'une quelconque des revendications 1 à 4 précédentes, dans lequel le groupe amino $R^1R^2N$- est le 1-phényléthylamino, le 1-(1-naphtyl)éthylamino, le 1-cyclohexyléthylamino ou le 1-(pyride-3-yl)éthylamino.

**9.** Composé selon la revendication 1 qui comprend l'une des caractéristiques suivantes;
(i) R représente un groupe méthyle;
(ii) A représente un groupe 3-pyridyle;
(iii) Y représente un groupe méthylène;
(iv) B représente un groupe alkyle inférieur ou un groupe phényle éventuellement substitué;
(v) $R^2$ représente un atome d'hydrogène;
(vi) $R^1$ représente un atome d'hydrogène, un groupe alkyle inférieur, portant éventuellement un substituant choisi parmi les groupes naphtyle et phényle ou 3-pyridyle éventuellement substitué, cycloalkyle et hydroxy, ou représente un groupe alcanoyle inférieur, aroyle ou arylsulfonyle.

**10.** Procédé de préparation d'un dérivé de thioformamide selon la revendication 1 ou d'un sel pharmaceutiquement acceptable de celui-ci, qui comprend:

(A) lorsque $R^2$ représente l'atome d'hydrogène, la réduction d'un composé de formule générale:

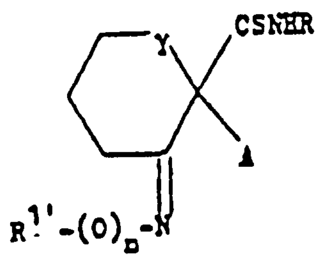

(III)

dans laquelle $R^{1'}$ est tel que défini dans la revendication 1 pour $R^1$ ou, lorsque n est 1, $R^{1'}$ peut représenter l'atome d'hydrogène;

(B) lorsque $R^1$ ou $R^2$, ou les deux, représente un groupe alkyle, cycloalkyle, cycloalkylalkyle, aryle, arylalkyle, aryloxyalkyle, hétérocyclylalkyle aromatique ou hétérocyclyloxyalkyle aromatique, éventuellement substitué, la réaction d'un composé de formule générale (I), dans laquelle l'un au moins des radicaux $R^1$ et $R^2$ représente l'atome d'hydrogène, avec l'halogénure d'alkyle, de cycloalkyle, de cycloalkylalkyle, d'aryle, d'arylalkyle, d'aryloxyalkyle, d'hétérocyclylalkyle aromatique ou d'hétérocyclyloxyalkyle aromatique correspondant, en l'absence ou en présence d'un solvant organique inerte;

(C) lorsque l'un des radicaux $R^1$ et $R^2$, ou les deux représente un groupe BC(=O)-, tel que défini dans la revendication 1, la réaction du composé de formule générale (I), dans laquelle l'un au moins des radicaux $R^1$ et $R^2$ représente un atome d'hydrogène, avec un halogénure d'acide d'un composé de formule générale:

BCOOH (IV)

ou un anhydride d'acide réactif de celui-ci;

(D) lorsque $R^1$ représente un groupe $BSO_2$-, tel que défini dans la revendication 1, la réaction d'un composé de formule générale (I), dans laquelle n est 0 et $R^1$ représente un atome d'hydrogène, avec un halogénure de sulfonyle de formule générale:

$BSO_2X^1$ (V)

dans laquelle B est tel que défini ci-dessus et $X^1$ représente un atome d'halogène;

éventuellement suivie de la transformation du dérivé de thioformamide ainsi obtenu en un sel pharmaceutiquement acceptable de celui-ci.

**11.** Composition pharmaceutique, qui comprend un dérivé de thioformamide selon la revendication 1 ou un sel pharmaceutiquement acceptable de celui-ci, en association avec un véhicule ou un enrobage pharmaceutiquement acceptable.

**12.** Dérivé de thioformamide selon la revendication 1 ou un sel pharmaceutiquement acceptable de celui-ci, pour l'utilisation comme médicament.

**Revendications pour les Etats contractants suivants : ES, GR**

**1.** Procédé de préparation d'un dérivé de thioformamide de formule générale:

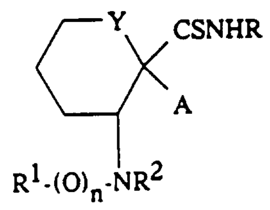

I

dans laquelle R représente un radical alkyle à chaîne droite ou ramifiée contenant de 1 à 4 atomes de carbone, A représente un radical hétérocyclique aromatique contenant un ou deux atomes d'azote (éventuellement substitués par un radical alkyle ou alcoxy à chaîne droite ou ramifiée contenant 1 à 4 atomes de carbone, ou par un atome d'halogène), choisi parmi le pyride-3-yle, l'isoquinoléine-4-yle, le tétrahydroquinoléine-3-yle, le quinoléine-3-yle, le pyridazine-4-yle, le pyrimide-5-yle, le thiazole-5-yle, le thiéno[2,3-b]pyridine-5-yle, le pyrazine-2-yle, l'indole-3-yle et le thiéno[2,3-b]pyridine-6-yle, ou représente un groupe phényle substitué en position 3 et/ou 5 par un groupe cyano, nitro, trifluorométhyle, carbamoyle, carboxy, alcanoyle en $C_2$-$C_5$,alcoxycarbonyle en $C_2$-$C_5$, alkylsulfonyle en $C_1$-$C_4$, ou un atome de fluor, de chlore ou de brome et, éventuellement, encore substitué par un ou des atome(s) d'halogène, un ou des groupe(s) alkyle en $C_1$-$C_4$ ou aryle en $C_6$-$C_{12}$, ou A est un groupe phényle non substitué ou substitué par un ou des atome(s) d'halogène, un ou des groupe(s) alkyle en $C_1$-$C_4$ ou aryle en $C_6$-$C_{12}$ ou par des substituants qui forment ensemble un noyau condensé, et Y représente une liaison de valence ou un radical méthylène ou éthylène, $R^2$ représente un atome d'halogène, un groupe alkyle, cycloalkyle, cycloalkylalkyle, aryle, arylalkyle, aryloxyalkyle, hétérocyclylalkyle aromatique ou hétérocyclyloxyalkyle aromatique, éventuellement substitué par un ou plusieurs atomes d'halogène, ou un groupe BC(=O)- dans lequel B représente un groupe alkyle, aryle ou un groupe hétérocyclique aromatique, éventuellement substitué par un ou plusieurs atomes d'halogène, ou B représente un radical benzyle, phénéthyle, 1-naphtylméthyle, 2-naphtylméthyle ou pyride-3-ylméthyle, n représente 0 ou 1, et, lorsque n représente 0, $R^1$ représente un atome d'hydrogène, un groupe alkyle, cycloalkyle, cycloalkylalkyle, aryle, arylalkyle, aryloxyalkyle, hétérocyclylalkyle aromatique ou hétérocyclyloxyalkyle aromatique, éventuellement substitué par un ou plusieurs substituants atomes d'halogène ou un groupe BC(=O)- ou $BSO_2$-, et, lorsque n représente 1, $R^1$ représente un radical alkyle à chaîne droite ou ramifiée contenant de 1 à 4 atomes de carbone qui est non substitué ou substitué par un ou plusieurs substituants choisis parmi les groupes alcényle en $C_2$-$C_4$, carboxy, alcoxycarbonyle en $C_2$-$C_5$, hydroxy, alcoxy en $C_1$-$C_4$, carbamoyle (non substitué ou substitué par un ou deux groupes alkyle en $C_1$-$C_4$), amino, alkylamino en $C_1$-$C_4$ et di-alkylamino en $C_1$-$C_4$, ou représente un radical benzyle, phénéthyle, 1-naphtylméthyle, 2-naphtylméthyle ou pyride-3-ylméthyle, chacun d'entre eux étant non substitué ou substitué sur le noyau par un ou plusieurs atomes d'halogène ou par les groupes hydroxy, alkyle en $C_1$-$C_4$, alcoxy en $C_1$-$C_4$ (le groupe alcoxy étant non substitué ou substitué comme défini pour les groupes alkyle représentés par $R^1$), cyano, nitro, trifluorométhyle, carboxy, alkylamino en $C_1$-$C_4$, alcanoylamino en $C_2$-$C_5$ ou alcoxycarbonyle en $C_2$-$C_5$, les groupes et les groupements aryle, dans la définition de $R^1$, $R^2$ et B, étant choisis parmi les groupes phényle et naphtyle, éventuellement substitués par un ou plusieurs substituants choisis parmi les atomes d'halogène, et les groupes et les groupements hétérocycliques aromatiques, dans la définition de $R^1$, $R^2$ et B, étant choisis parmi le groupe pyride-3-yle et des sels pharmaceutiquement acceptables de ce composé, lequel procédé comprend:

(A) lorsque $R^2$ représente l'atome d'hydrogène, la réduction d'un composé de formule générale:

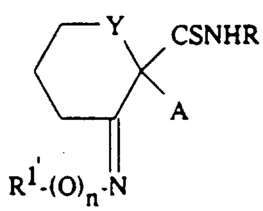

(III)

dans laquelle $R^{1'}$ est tel que défini ci-dessus pour $R^1$ ou, lorsque n est 1, $R^{1'}$ peut représenter l'atome d'hydrogène;

(B) lorsque $R^1$ ou $R^2$, ou les deux, représente un groupe alkyle, cycloalkyle, cycloalkylalkyle, aryle, arylalkyle, aryloxyalkyle, hétérocyclylalkyle aromatique ou hétérocyclyloxyalkyle aromatique éventuellement substitué, la réaction d'un composé de formule générale (I), dans laquelle l'un au moins des radicaux $R^1$ et $R^2$ représente l'atome d'hydrogène, avec l'halogénure d'alkyle, de cycloalkyle, de cycloalkylalkyle, d'aryle, d'arylalkyle, d'aryloxyalkyle, d'hétérocyclylalkyle aromatique ou d'hétérocyclyloxyalkyle aromatique correspondant, en l'absence ou en présence d'un solvant organique inerte;

(C) lorsque l'un des radicaux $R^1$ et $R^2$, ou les deux, représente un groupe BC(=O)-, tel que défini ci-dessus, la réaction du composé de formule générale (I), dans laquelle l'un au moins des radicaux $R^1$ et $R^2$ représente un atome d'hydrogène, avec un halogénure d'acide d'un composé de formule générale:

BCOOH (IV)

ou un anhydride d'acide réactif de celui-ci;
(D) lorsque $R^1$ représente un groupe $BSO_2$-, tel que défini ci-dessus, la réaction d'un composé de formule générale (I), dans laquelle n est 0 et $R^1$ représente un atome d'hydrogène, avec un halogénure de sulfonyle de formule générale:

$BSO_2X^1$ (V)

dans laquelle B est tel que défini ci-dessus et $X^1$ représente un atome d'halogène; éventuellement suivie de la transformation du dérivé de thioformamide ainsi obtenu en un sel pharmaceutiquement acceptable de celui-ci.

**2.** Procédé selon la revendication 1, dans lequel Y représente le groupe méthylène.

**3.** Procédé selon la revendication 1 ou 2, dans lequel A représente un groupe 3-pyridyle, 6-chloropyride-3-yle, 5-bromopyride-3-yle, 3-quinoléinyle, 4-isoquinoléinyle ou 5-pyrimidyle, ou un groupe phényle substitué en position 3 et/ou 5 par un groupe attracteur d'électrons choisi parmi les groupes cyano, nitro, trifluorométhyle, carbamoyle, carboxy, alcanoyle en $C_2$-$C_5$, alcoxycarbonyle en $C_2$-$C_5$ ou alkylsulfonyle en $C_1$-$C_4$, ou un atome de fluor, de chlore ou de brome et, éventuellement, encore substitué par un ou des atome(s) d'halogène, un ou des groupe(s) alkyle en $C_1$-$C_4$ ou aryle en $C_6$-$C_{12}$, ou le groupe phényle A peut être substitué par un ou des atome(s) d'halogène, un ou des groupe(s) alkyle en $C_1$-$C_4$ ou aryle en $C_6$-$C_{12}$, ou par des substituants qui forment ensemble un noyau condensé.

**4.** Procédé selon l'une quelconque des revendications précédentes, dans lequel A représente un groupe phényle, 4-chlorophényle, 3-trifluorométhylphényle, 3-nitrophényle, 3,4-dichlorophényle ou 2-naphtyle.

**5.** Procédé selon l'une quelconque des revendications précédentes dans lequel B représente un groupe phényle ou pyride-3-yle ou un groupe alkyle à chaîne droite ou ramifiée (contenant 1 à 6 atomes de carbone), chacun d'entre eux étant éventuellement substitué par un ou plusieurs atomes d'halogène, ou B représente un radical benzyle, phénéthyle, 1-naphtylméthyle, 2-naphtylméthyle ou pyride-3-ylméthyle.

**6.** Procédé selon l'une quelconque des revendications précédentes, dans lequel B représente un groupe phényle, 4-fluorophényle, 3,4-difluorophényle, pyride-3-yle, méthyle, butyle, benzyle ou phénéthyle.

**7.** Procédé selon l'une quelconque des revendications 1 à 4 précédentes, dans lequel $R^2$ représente un atome d'hydrogène.

**8.** Procédé selon l'une quelconque des revendications 1 à 4 précédentes, dans lequel le groupe amino $R^1R^2N$- est le 1-phényléthylamino, le 1-(1-naphtyl)éthylamino, le 1-cyclohexyléthylamino ou le 1-(pyride-3-yl)éthylamino.

**9.** Procédé selon la revendication 1, qui comprend l'une des caractéristiques suivantes;
(i) R représente un groupe méthyle;
(ii) A représente un groupe 3-pyridyle;
(iii) Y représente un groupe méthylène;
(iv) B représente un groupe alkyle inférieur ou un groupe phényle éventuellement substitué;
(v) $R^2$ représente un atome d'hydrogène;
(vi) $R^1$ représente un atome d'hydrogène, un groupe alkyle inférieur, portant éventuellement un substituant choisi parmi les groupes naphtyle et phényle ou 3-pyridyle éventuellement substitué, cycloalkyle et hydroxy, ou représente un groupe alcanoyle inférieur, ou aroyle ou arylsulfonyle.